# EUROPEAN PATENT SPECIFICATION

(11) **EP 1 399 480 B1**
(45) Date of publication and mention of the grant of the patent: **17.01.2007**
(21) Application number: 02740899.6
(22) Date of filing: 19.06.2002
(51) Int. Cl.: C07K 14/705, C12N 5/10, C12N 15/12, A61K 38/17, A61K 48/00, A61P 37/06

(54) **MULTIMERS OF CLASS I MAJOR HISTOCOMPATIBILITY COMPLEXES CONTAINING MODIFIED BETA2-MICROGLOBULIN PROTEINS**
MULTIMERE DES HAUPT-HISTOKOMPATIBILITÄTS-KOMPLEXES DER KLASSE I, DIE MODIFIZIERTEN BETA2-MIKROGLOBULIN ENTHALTEN
MULTIMERS DES COMPLEXES MAJEURS D'HISTOCOMPATIBILITE DE CLASSE I CONTENANT DES PROTEINES BETA2-MICROGLOBULIN MODIFIEES

(30) Priority: 20.06.2001 GB 0115071
(43) Date of publication of application: 24.03.2004
(73) Proprietor: Avidex Limited, Oxon OX14 4RX (GB)
(72) Inventor: JAKOBSEN, Bent, Karsten, Avidex Limited, Oxon OX14 4RX (GB); BOULTER, Jonathan Michael Avidex Limited, Oxon OX14 4RX (GB)
(74) Representative: Walls, Alan James
(86) International application number: PCT/GB2002/002866
(87) International publication number: WO 2002/102840

(56) References cited:
- WO-A-01/44296
- WO-A-01/72768
- WO-A-99/21576
- WO-A-02/074331
- SYLVESTER-HVID C ET AL: "A single-chain fusion molecule consisting of peptide, major histocompatibility gene complex class I heavy chain and beta2-microglobulin can fold partially correctly, but binds peptide inefficiently." SCANDINAVIAN JOURNAL OF IMMUNOLOGY, vol. 50, no. 4, October 1999 (1999-10), pages 355-362, XP002240676 ISSN: 0300-9475
- ALTMAN J ET AL: "Phenotypic analysis of antigen-specific T lymphocytes" SCIENCE, AMERICAN ASSOCIATION FOR THE ADVANCEMENT OF SCIENCE, US, vol. 274, no. 5284, 4 October 1996 (1996-10-04), pages 94-96, XP002135711 ISSN: 0036-8075
- DANIELS MARK A ET AL: "Critical role for CD8 in T cell receptor binding and activation by peptide/major histocompatibility complex multimers." JOURNAL OF EXPERIMENTAL MEDICINE, vol. 191, no. 2, 17 January 2000 (2000-01-17), pages 335-345, XP002240677 ISSN: 0022-1007
- GLICK MEIR ET AL: "Novel CD8+ T cell antagonists based on beta2-microglobulin." JOURNAL OF BIOLOGICAL CHEMISTRY, vol. 277, no. 23, 7 June 2002 (2002-06-07), pages 20840-20846, XP002240678 June 7, 2002 ISSN: 0021-9258

## Description

The present invention relates to multimers of Class I Major Histocompatibility Complexes (MHC) containing modified β₂-microglobulin (β₂m) proteins wherein the mutated residue is one or more of Serine₅₇, Lysine₅₈, Aspartate₅₉, Tryptophan₆₀ and Serine₆₁, and to their use in immunosuppressive therapy, in particular as inhibitors of cytotoxic T cell responses.

MHC molecules are specialised protein complexes which present short protein fragments (peptide antigens) on the cell surface for recognition by the cellular arm of the adaptive immune system.

Class I MHC is a dimeric protein complex consisting of a variable heavy chain and a constant light chain, β₂-microglobulin. Class I MHC presents peptides which are processed intracellularly, loaded into a binding cleft in the MHC, and transported to the cell surface where the complex is anchored in the membrane by the MHC heavy chain. Peptides are usually 8-11 amino acids in length, depending on the degree of arching introduced in the peptide when bound in the MHC. The binding cleft which is formed by the membrane distal α1 and α2 domains of the MHC heavy chain has "closed" ends, imposing quite tight restrictions on the length of peptide which can be bound.

β₂-microglobulin is a polypeptide which is found free in serum, which is non-covalently associated with MHC Class I molecules at the cell surface and which can exchange in the MHC complex with other free β₂m molecules (Bemabeu, *et al. Nature* **308:** 642-5 (1984); Cook, *et al. JImmunol 157:* 2256-61 (1996); Horig, *et al. Proc Natl Acad Sci U S A* **94**: 13826-31 (1997); Hyafil & Strominger, *Proc Natl Acad Sci U S A* 76: 5834-8 (1979); Luscher, *et al. J Immunol* **153**: 5068-81 (1994); Parker, *et al. J Immunol* **149**: 1896-904 (1992); Smith, *et al. Proc Natl Acad Sci U S A* **89**: 7767-71 (1992)). Unlike MHC heavy chain polypeptides, β₂m is not anchored in the cell membrane. β₂m has the structure of an immunoglobulin C (constant) domain and also associates with a number of other class I-related structures, such as the products of the CD1 genes in man.

A wide spectrum of cells can present antigen, as MHC-peptide, and the cells which have that property are known as antigen presenting cells (APCs). The type of cell which presents a particular antigen depends upon how and where the antigen first encounters cells of the immune system. APCs include the interdigitating dendritic cells found in the T cell areas of the lymph nodes and spleen in large numbers; Langerhan's cells in the skin; follicular dendritic cells in B cell areas of the lymphoid tissue; monocytes, macrophages and other cells of the monocyte/macrophage lineage; B cells and T cells; and a variety of other cells such as endothelial cells and fibroblasts which are not classical APCs but can act in the manner of an APC.

APCs are recognised by a subgroup of lymphocytes which mature in the thymus (T cells) where they undergo a selection procedure ensuring that T cells which respond to self-peptides are eradicated (negative selection). In addition, T cells which do not have the ability to recognise the MHC variants which are presented (in man the HLA haplotypes) fail to mature (positive selection).

Recognition of specific MHC-peptide complexes by T cells is mediated by the T cell receptor (TCR) which consists of an α- and a β chain, both of which are anchored in the membrane. In a recombination process similar to that observed for antibody genes, the TCR α- and β- genes rearrange from Variable, Joining, Diversity and Constant elements creating enormous diversity in the extracellular antigen binding domains (10¹³ to 10¹⁵ different possibilities).

Antibody receptors and TCRs are the only two types of molecules which recognise antigens in a specific manner, and thus the TCR is the only receptor specific for particular peptide antigens presented in MHC, the alien peptide often being the only sign of an abnormality within a cell.

The vast majority of T cells restricted by Class I MHC-peptide complexes also require the engagement of the coreceptor CD8 for activation, while T cells restricted by Class II MHC require the engagement of CD4. The exact function of the coreceptors in T cell activation is not yet entirely clarified; neither are the critical mechanisms and parameters controlling activation. However, both CD8 and CD4 have cytoplasmic domains which are associated with the kinase p56 ^{lck} which is involved in the very earliest tyrosine phosphorylation events which characterise T cell activation. CD8 is a dimeric receptor, expressed either in an αα form or, more commonly, in an αβ form. CD4 is a monomer. In the CD8 receptor, only the α- chain is associated with p56^{lck}.

Recent determinations of the physical parameters controlling binding of TCR and CD8 to MHC, using soluble versions of the receptors, have shown that binding by TCR dominates the recognition event. TCR has significantly higher affinity for MHC than the coreceptors.

The individual interactions of the receptors with MHC are very short-lived at physiological temperature, i.e. 37°C. An approximate figure for the half life of a TCR-MHC/peptide interaction, measured with a human TCR specific for the influenza virus "matrix" peptide presented by HLA-A*0201 (HLA-A2), is 0.7 seconds. The half life of the CD8αα interaction with the MHC/peptide complex is less than 0.01 seconds or at least 18 times faster (Willcox, *et al. Immunity* **10**: 357-65 (1999); Wyer, *et al. Immunity* **10:** 219-225 (1999)).

Class I MHC restricted cellular immune responses are mediated by cytotoxic T cells (CTLs). CTLs are activated through protein-protein interactions between T cell receptors (TCRs) and their coreceptors, CD8 molecules, on the T cell and MHC/peptide ligands on the target cell surface. TCRs form contacts both to the antigen peptide and to the heavy chain of the MHC complex *(Ding, et al. Immunity* **11**: 45-56 (1999); Ding, *et al. Immunity* **8:** 403-11 (1998); Garboczi, *et al. Nature* **384:** 134-41 (1996); *Garcia, et al. Science* **274:** 209-19 Issn: 0036-8075 (1996); and provide the antigen specificity of the T cell. CD8 binds to the heavy chain and the light chain (β₂-microglobulin) of the MHC complex, but not to the antigen peptide (Gao, *et al. Nature* **387:** 630-4 (1997)). WO 99/21576 details the production of soluble human CD8 molecules, capable of binding to peptide-MHC molecules, suitable for biochemical studies and therapeutic applications. These protein binding events lead to activation of signal transduction in the T cell.

Single-chain fusion proteins consisting of an antigenic peptide linked to a murine Class I MHC heavy chain molecule which is in turn linked to a β2-microglobulin have been expressed in bacterial systems. (Sylvester-Hvid *et al.,* (1999) *Scand J. Immunol* **50**: 355-362) Serological and functional peptide analysis of the construct detailed in this study suggest that it was at least partially correctly refolded.

WO 01/72768 details the bacterial production of single-chain chain fusion proteins comprising a human Class I MHC heavy chain linked to a β2-microglobulin. These constructs are shown to be capable of loading antigenic peptides.

A further study *(Daniels et al.,* (2000) *J. Exp Med* **191** (2): 335-345) utilised recombinant peptide-Class I MHC multimers to investigate the importance of coreceptor (CD8) binding in the engagement of T cells to such molecules. The study concluded that the engagement and activation of T cells by such multimers was highly CD8 dependent.

In contrast to antibody-antigen interactions, TCR-MHC/peptide interactions and, in particular, CD8-MHC interactions are characterised by low affinities and fast kinetics (Willcox, *et al. Immunity* **10:** 357-65 (1999); *Wyer, et al. Immunity* **10:** 219-225 (1999)). The interactions between T cells and antigen presenting cells are believed to be stabilised by multiple simultaneous receptor-ligand contacts, increasing the avidity of cell-cell interactions (Mescher, *Immunol Rev 146,* 177-210 (1995); Norment, *et al. Nature* **336:** 79-81 (1988)).

Cytotoxic T cells are exquisitely sensitive to interference that disrupts the normal kinetics and/or affinities of the interactions required for activation. For example, altered peptide ligands (APLs) presented together with the normal antigen peptide on the MHC molecules of the antigen presenting cell can cause a failure to activate CTLs. Remarkably, this phenomenon can, in some cases, be observed even when the antagonist APL is presented at significantly lower molar ratios than the normal antigen (Bertoletti, *et al. Nature* **369:** 407-10 Issn: 0028-0836 (1994); Klenerman, *et al. Nature* **369:** 403-7 (1994); Purbhoo, *et al. Proc. Natl. Acad. Sci. USA* **95:** 4527-4532 (1998)). Thus, CTLs are highly sensitive to variations in TCR-antigen contacts, even if the majority of these are within the 'normal' parameters required for activation.

Suppressors of the cellular arm of the immune system, such as suppressors of CD4 or CD8 T cells, are urgently needed for the treatment of auto-immune disorders, such as rheumatoid arthritis, lupus erthymatosus, psoriasis vulgaris, ankylosing spondylitis, Reiter's disease, post-salmonella arthritis, post-shigella arthritis, post-yersinia arthritis, post-gonococcal arthritis, uveitis, amylodosis, idiopathic hemachromatosis and myasthenia gravis, of hypersensitivity (such as allergic reaction), and the prevention of graft rejection and graft-versus-host disease. Antibodies directed against CD4 and CD8 have been tried (De Fazio, *et al. Transplantation* **61:** 104-10 (1996)), but with limited success and antibodies in general are not well suited as drugs since they tend to induce secondary immune responses and are short-lived. Administration of steroids is another way of suppressing the immune system but their effect is extremely indirect and associated with severe side-effects.

It is known that class I MHC/peptide complex tetramers, which have been modified so that the heavy chain of the MHC can no longer bind CD8, have the ability to cause apoptosis of CD8⁺ T cells with minimal cellular activation of the T cell. The MHC molecules have mutations in the α3 domains of the heavy chain (Xu *et al.,* 2001, *Immunity,* **14**:591-602) so that CD8 binding thereto is inhibited. The applicants copending application (WO 01/44296) details the production of mutated β2-microglobulin molecules and the use thereof to inhibit the binding of CD8+ T cells to MHC molecules comprising such mutated β2-microglobulins.

The present invention aims to provide an alternative means of disrupting CD8⁺ T cell responses.

According to a first aspect of the present invention, there is provided a multimer comprising a plurality of soluble class I Major Histocompatibility Complexes (MHCs) at least one of which has a modified β₂-microglobulin whose binding to CD8 as measured by surface plasmon resonance is inhibited, wherein the mutated residue is one or more of Serine₅₇, Lysine₅₈, Aspartate₅₉, Tryptophan₆₀ and Serine₆₁, said multimer being capable of inhibiting a CD8+T cell response.

Multimers of the present invention are useful in an in-vitro method of inhibiting a CD8⁺ T cell response, comprising exposing the CD8⁺ T cell to a multimer comprising a plurality of class I Major Histocompatibility Complexes (MHCs) at least one of which has a modified β₂-microglobulin whose binding to CD8 as measured by surface plasmon resonance is inhibited, wherein the mutated residue is one or more of Serine₅₇, Lysine₅₈, Aspartate₅₉, Tryptophan₆₀ and Serine₆₁.

In other aspects, the invention provides a multimer of the invention for use in medicine, and in the manufacture of a medicament for inhibiting CD8⁺ T cell response.

As used herein "inhibited", when used in the context of the binding of one entity to another, means that the binding is prevented altogether or reduced to such a level that the normal physiological results of binding cannot be observed or are not significant. Even low levels of binding inhibition can have severe physiological effects (for example, low levels of soluble CD8 receptor can cause CTL inhibition *(Sewell, et al. Nature Medicine* **5**: 399-404 (1999)). It would be expected that, *in vitro,* the binding of soluble CD8 molecule to a soluble HLA complex refolded with a modified β₂-microglobulin may be inhibited by at least 20%, and more likely 50 to 100%.

The main utility of the present invention is in the treatment of humans. Thus, the MHCs are preferably Human Leukocyte Antigen (HLA) types. However, it is to be understood that the present invention finds use in the treatment of non-human animals.

In a preferred embodiment, the present invention relates to a means of inhibiting CD8⁺ T cell responses which is completely different to those proposed hitherto, in that CTLs bind peptide-MHC while being inhibited from activating by inhibiting the binding of CD8 on the T cell surface with the MHC molecules. This is achieved by providing multimers of MHC complexes of the invention having β₂-microglobulin which has been modified such that its binding to CD8 is impaired or inhibited. This has the advantage over the MHC tetramers proposed by Xu *et al., (Immunity,* 2001, **14**:50-602) that a series of different MHC complexes which have a reduced ability to bind CD8 can be produced using only a single modified β₂m rather than a series of different modified MHC heavy chains.

Stimulation of CD8⁺ T cells by specific peptide-MHC antigen results in activation and proliferation. Activated CD8⁺ T cells produce a variety of molecules, some of which stimulate a more general immune response (eg. IFN-γ), and some of which lyse the target cell (eg. perforin). CD8⁺ T cells activated by either antigen presenting cells, or by multimeric peptide-MHC complexes, also undergo activation-induced cell death (AICD). This is may be a necessary control pathway to prevent excessive clonal expansion of activated T cells. AICD is mediated by the interaction of Fas and Fas-ligand. Fas-ligand is expressed on the surface of activated T cells and engages with Fas-ligand on antigen presenting cells, or even on other T cells, to initiate AICD.

The present invention is predicated on the finding that multimers of MHC having β₂-microglobulin with an inhibited ability to bind CD8 cause apoptosis but fail to cause activation, suggesting that the signal produced by CD8 binding to peptide-MHC is not required for initiation of AICD. Although it is preferred if each MHC of the multimers of the present invention has a modified β₂m whose binding to CD8 as measured by surface plasmon resonance is inhibited, wherein the mutated residue is one or more of Serine₅₇, Lysine₅₈, Aspartate₅₉, Tryptophan₆₀ and Serine₆₁, it is to be understood that the present invention encompasses MHC multimers in which not all of the MHC molecules have such a modified β₂m.

Multimers of the invention comprising class I MHCs containing mutant β₂m proteins may be made using specific class I MHC alleles and specific peptide. This allows the targeted inhibition of populations of T cells which are activated against specific epitopes, which may be associated with a specific disorder. Peptide-specific multimeric class I MHC complexes of the invention containing mutant β₂m protein may be produced using synthetic peptide (O'Callaghan, *et al. Anal Biochem* **266**(1): 9-15 (1999)). Alternatively, the specific peptide may be expressed as a fusion protein with the mutant β₂m with a linker between the peptide and the β₂m to allow the peptide to sit properly in the peptide-binding groove of the class I MHC complex. Multimeric complexes made in this way will specifically bind to T cells which are activated in response to the particular peptide-MHC antigen. Binding of these multimeric complexes will induce apoptosis, but, because the signal caused by CD8 binding is reduced, the T cells involved will not activate. Such MHC-peptide complexes are useful for the treatment of specific diseases associated with specific peptides or HLA types. Table 1 shows conditions and their association with HLA allele.

**Table 1. Association of HLA with disease**

| **Disease** | | **HLA allele** | **Relative risk** |
|---|---|---|---|
| (a) | Class I, HLA-27 associated | | |
| | Ankylosing spondylitis | B27 | 87.4 |
| | Reiter's disease | B27 | 37.0 |
| | Post-salmonella arthritis | B27 | 29.7 |
| | Post-shigella arthritis | B27 | 20.7 |
| | Post-yersinia arthritis | B27 | 17.6 |
| | Post-gonococcal arthritis | B27 | 14.0 |
| | Uveitis | B27 | 14.6 |
| | Amyloidosis in rheumatoid arthritis | B27 | 8.2 |

| (b) | Other Class I associations | | |
|---|---|---|---|
| | Subacute thyroiditis | Bw35 | 13.7 |
| | Psoriasis vulgaris | Cw6 | 13.3 |
| | Idiopathic hemochromatosis | A3 | 8.2 |
| | Myasthenia gravis | B8 | 4.4 |

| | | | |
|---|---|---|---|
| *(Data from Ryder et al. HLA and disease Registry 1979. Tissue Antigens, supplement 1979)* | | | |

Alternatively, non-peptide specific multimeric class I MHC complexes of the invention containing mutant β₂m may be made by using a pool of random peptides during the production of the class I MHC complexes. Such MHC-specific multimers are useful in the more general treatment of allo-reactive conditions such as graft rejection and graft-versus-host disease (Xu, *et al. Immunity* **14:** 591-602 (2001)). Alternatively, certain positions of the peptides may be randomised so as to make it more likely that antigen complexes are included that bind to a larger proportion of T cells. Because of the high degree of variation in these peptides, T cell recognition will not be dominated by a subset of T cells which are specific for one particular antigen. Instead, the recognition will be dominated by the bias towards the particular HLA molecule presenting the redundant antigens. Non-peptide specific multimeric class I MHC complexes containing mutant β₂m may be made with peptide which consists of small side-chain residues (such as alanine) except at the anchor positions. Such peptides can bind to the class I MHC complex, but cannot interact significantly with the T cell receptor because they do not have any amino acid side chains (except hydrogen) which could contribute to T cell binding. Allo-reactive responses are thought to be dominated by the interactions between the T cell receptor and the MHC component of the peptide-MHC complex. Therefore, such multimeric class I MHC complexes containing mutant β₂m may be particularly useful for treatment of allo-reactive conditions, such as graft rejection. Thus, the multimeric MHC of the present invention may comprise a peptide selected from a random pool of peptides, or which presents substantially no T cell recognition features or in which one or more T cell recognition features are randomly present.

Methods for the production of MHC multimers are known. Soluble class I MHC-peptide complexes were first obtained by cleaving the molecules of the surface of antigen presenting cells with papain (Bjorkman, *et al, J Mol Biol* (1985) **186**: 205-10). Although this approach provided material for crystallisation, in recent years it has been replaced for class I molecules by individual expression of heavy and light chains in *E. coli,* followed by refolding in the presence of synthetic peptide (Gao, *et al, Prot. Sci.* (1998) 7: 1245-49; *Gao, et al, Nature* (1997) **387**: 630-4; Garboczi, *et al, Proc Natl Acad Sci USA* (1992) **89:** 3429-33 Issn: 0027-8424; Garboczi, *et al, J Mol Biol* (1994) **239:** 581-7 Issn: 0022-2836; Madden, *et al,* [published erratum appears in *Cell* (1994) Jan 28;**76**(2):following 410]. *Cell* (1993) **75:** 693-708 Issn: 0092-8674; Reid, *et al, J Exp Med* (1996) **184:** 2279-86; Reid, *et al, FEBS Lett* (1996) **383:** 119-23; Smith, *et al, Immunity* (1996) **4:** 215-28 Issn: 1074-7613; Smith, *et al, Immunity* (1996) **4:** 203-13 Issn: 1074-7613). This approach has several advantages over previous methods; higher yields are obtained at a lower cost, peptide identity can be accurately controlled, and the final product is more homogeneous. Furthermore, expression of modified heavy or light chains, for instance fused to a protein tag, can be easily achieved.

In the present invention, it is preferred if two or more MHC molecules are linked (covalently or otherwise) together to form a multivalent complex. The MHC molecules may be associated with one another via a linker molecule. Alternatively or additionally, individual MHC molecules may be linked by being attached to larger entities, such as liposomes or particles to form multimers of the invention.

Suitable linker molecules include multivalent attachment molecules such as avidin, streptavidin and extravidin, each of which has four binding sites for biotin. Thus, biotinylated MHC molecules can be formed into multimeric complexes of MHC. The number of MHC molecules in the resulting complex will depend upon the quantity of MHC in relation to the quantity of linker molecule used to make the complexes, and also on the presence or absence of any other biotinylated molecules. Preferred complexes are trimeric or tetrameric MHC complexes. Alternative linker molecules include IgM, human complement factor C1Q and haemoglobulin tetramerisation domain-derived molecules.

Suitable structures for attachment of MHC, optionally already in multimeric form, include liposomes and solid structures, which are preferably particles such as beads. Other structures known to those skilled in the art which may be externally coated with MHC molecules are also suitable.

The production of tetrameric molecules of peptide-MHC complexes is described in Altman, *et al, Science* (1996) **274**:94-6. The higher avidity of the multimeric interaction provides a dramatically longer half-life for the molecules binding to a T cell than would be obtained with binding of a monomeric peptide-MHC complex.

The tetrameric peptide-MHC complex may be made with synthetic peptide, mutated β2microglobulin (usually expressed in *E.coli),* and soluble MHC heavy chain (also expressed in *E.coli).* The transmembrane domain is truncated from the heavy chain and replaced with a protein tag constituting a recognition sequence for the bacterial enzyme BirA (Barker & Campbell, *J Mol Biol* (1981) **146:** 451-67; Barker & Campbell, *J Mol Biol* (1981) **146:** 469-92; Schatz, *Biotechnology N Y* (1993) **11:** 1138-43). Bir A catalyses the biotinylation of a lysine residue in a somewhat redundant recognition sequence (Schatz, *Biotechnology N Y* (1993) **11:** 1138-43). However, the specificity is high enough to ensure that the vast majority of protein will be biotinylated only on the specific position on the tag. The biotinylated protein can then be covalently linked to streptavidin which has four binding sites resulting in a tetrameric molecule of peptide-MHC complexes (Altman, *et al, Science* (1996) **274**:94-6). The β₂-m mutant proteins may have substitutions or deletions at any one or more of Serine₅₇, Lysine₅₈, Aspartate₅₉, Tryptophan₆₀ and Serine₆₁ and/or insertion mutations immediately before or after any of said residues which cause the binding of CD8 to be inhibited. Substitution mutations are preferred as they create minimal disruption to the structure of β₂m. The mutated residues, for instance sterically and/or electrostatically, prevent or impair the binding of CD8 to MHC complexes involving the mutant β₂-microglobulin proteins.

β₂-microglobulin is a soluble molecule that can be expressed as a recombinant protein (Gao, *et al. Prot. Sci.* **7**: 1245-49 (1998); Garboczi, *et al. Proc Natl Acad Sci U S A* **89***:* 3429-33 Issn: 0027-8424 (1992); Parker & Wiley, *Gene* **83**: 117-24 (1989)). It is preferred if the modified β₂-microglobulin is derived from human β₂-microglobulin. The sequence for this protein is known (Swissprot P01884 EMBL/Genbank M17986). However, the modified β₂-microglobulin may be derived from any species, e.g. dog, cat, rat or mouse. The sequences for rat and mouse β₂-microglobulin are known (Rat: Swissprot P07151, EMBL/Genbank Y00441, Mouse: Swissprot P01887 EMBL/Genbank X01838). For those β₂-microglobulins which have not be crystallised and hence do not have data available as to which residues thereof are involved in binding CD8, the skilled person can identify likely candidate residues for mutation using homology studies.

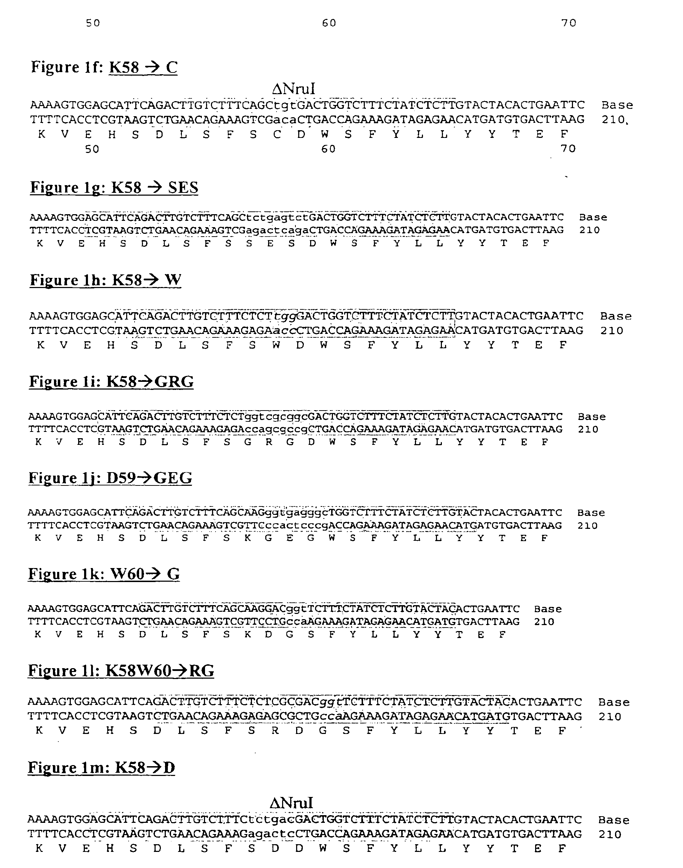

Unless the context dictates otherwise, reference to "human β₂m" herein means a protein comprising amino acid sequence 1-99 in Figure 1a, and reference to numbered amino acid residues in human β₂m is in accordance with the numbering of the amino acid residues in Figure 1a.

Binding of the CD8αα homodimer to the class I HLA complex is asymmetric, the two subunits of CD8 being involved in different contacts. Only one of the CD8 subunits is involved in binding β₂m, through its β strand 'A' in the immunoglobulin structure. The contributions of the β₂m-CD8α contacts to binding have not been established. However, considering the low affinity of CD8αα binding to HLA complex *(Wyer, et al. Immunity* **10**: 219-225 (1999)), it is likely that disruption of any contacts will lead to a significant inhibition of coreceptor's contribution to signal transduction in T cells.

Two residues in human β₂-microglobulin are the preferred targets for mutation. These are Lysine₅₈ and Tryptophan₆₀, both of which are involved in contacts with CD8 (Gao, *et al. Nature* **387:** 630-4 (1997)). Lysine₅₈ is the most preferred target. CD8 fits tightly into the binding 'cavity' formed by the heavy chain/light chain complex (Gao, *et al. Nature* **387:** 630-4 (1997)). In the CD8αα-HLA-A2 crystal structure, Lysine₅₈ protrudes from the β₂m loop towards the cavity occupied by CD8 (Gao, *et al. Nature* **387:** 630-4 (1997)) and forms contacts to the CD8 residues Arginine₄, Valine₂₄, Leucine₂₅, Leucine₂₆ and Aspartate₇₅. Therefore, amino acids with larger side chains, for instance arginine or tyrosine, at position 58 of β₂-microglobulin have severe effects on CD8 binding. Aspartic acid or glutamic acid residues may also be used for substituting Lysine₅₈, since these exercise electrostatic repulsion of CD8. Even fairly inconspicuous residues inserted in place of Lysine₅₈, for example serine, abrogate CD8 binding due to loss of specificity. In addition, CD8 binding may be inhibited by deleting Lysine₅₈ or inserting one or more resides which disrupt the cavity occupied by CD8.

It is known that mutations in the β₂m polypeptide can severely impair its ability to exchange into the HLA complex. One residue, mutation of which was found to impair the ability of the protein to exchange, was Tryptophan₆₀ (Fukazawa, *et al. J Immunol* **153**: 3543-50 (1994)). However, Lysine₅₈ is located in a loop, not a strand, of β₂m and is not involved in contacts with the class I heavy chain. Therefore amino acid substitutions of Lysine₅₈ should not impair the ability of the protein to exchange into the HLA complex. Lysine₅₈ is therefore the prime candidate residue for substitutions designed to generate a soluble protein, that can exchange with β₂m on the cell surface and serve to inhibit CTL activation by impairing CD8 contacts to the MHC complex.

The preferred mutations of Lysine₅₈ and the rationale for how they might change CD8 recognition, and inhibit this, are as follows:
Lysine₅₈ → Arginine (R). Arginine has a larger, and different, side chain than lysine, and therefore this substitution is likely to sterically hinder CD8 from slotting correctly into the cavity formed by the HLA complex.
Lysine₅₈ → Glutamate (E) or Aspartate (D). The side chains of glutamic acid and aspartic acid are oppositely charged to lysine, and therefore this substitution is likely to electrostatically repel CD8 binding to HLA complexes.
Lysine₅₈ → Serine (S). The side chain of serine lacks the positive charge of the lysine side chain. The lack of the electrostatic interactions to CD8 in which
Lysine₅₈ is involved is probably sufficient to weaken CD8 binding to HLA complexes.
Lysine₅₈ → Tyrosine (Y). Tyrosine has a substantially larger, and under normal conditions uncharged, side chain than lysine. This substitution is likely to sterically hinder CD8 binding to HLA complexes.
Lysine₅₈ → Cysteine (C). Mutation to cysteine creates a 'free' SH group on the surface of β₂m being contacted by CD8. This may in itself inhibit CD8 contacts, but the residue could also be used to derivatise the β₂m polypeptide.

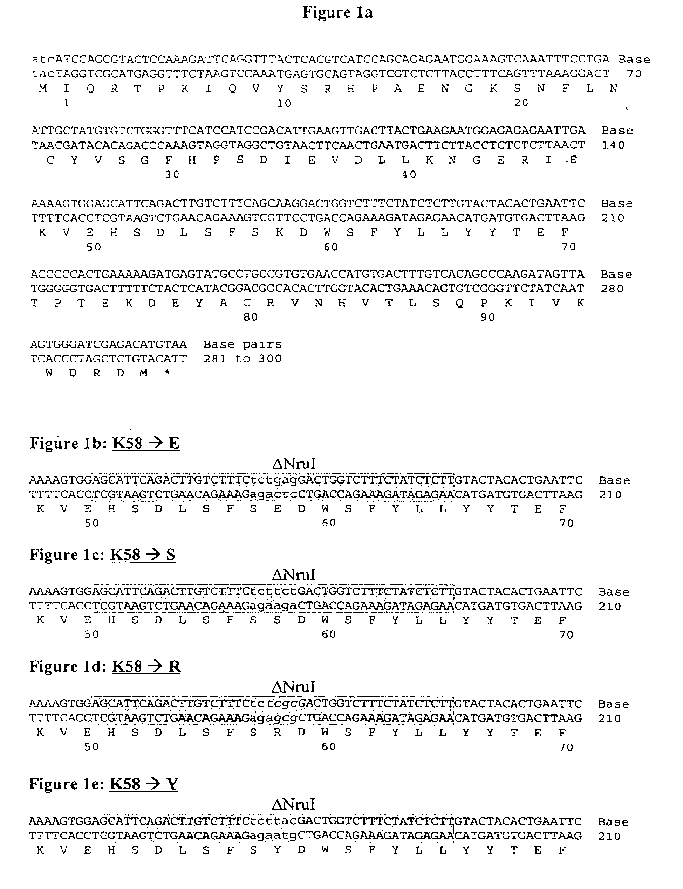

Figure 1a of the accompanying drawings shows the amino acid sequence of the processed human β₂m polypeptide and the DNA sequence encoding it, and Figures 1 b-m show the modifications that need to be made to this DNA sequence to introduce the mutations described above, as well as other mutations, and the amino acid sequences of the relevant regions of resulting β₂m proteins.

Tryptophan₆₀ is another preferred residue for mutation as it is involved in forming a contact to Arginine₄ in CD8α (Gao *et al., Nature* **387**:630-4 (1997)). However, data published in Fukazawa, *et al. J Immunol* **153**: 3543-50 (1994) suggests that mutation of this residue may interfere with the ability of the mutant protein to exchange. Thus, it is preferred that such mutant proteins are exposed to antigen presenting cells by means other than exchange, such as by expression of the mutant protein in the cell.

In addition to Lysine₅₈ and Tryptophan₆₀, because of the low affinity and transient nature of CD8-MHC interactions, functionally significant effects may be achieved by quite a large number of mutations (whether they be insertion, deletion or substitution mutations) in residues 57-61 of human β₂m (i.e. Serine₅₇, Aspartate₅₉ and Serine₆₁) either singly or in combination.

Substitution mutations of these residues can be divided into categories as follows:
- Mutation to Arginine, Histidine, Tryptophan, Tyrosine or Phenylalanine. These residues have large side chains and are therefore likely to cause steric obstruction of CD8 binding if introduced into the loop contacted by the coreceptor.
- Mutation to Glutamate or Aspartate. These residues have negatively charged side chains and are likely to cause electrostatic repulsion of CD8 binding, since Aspartate₇₅ of human CD8α is involved in contacts to β₂m (Gao, *et al. Nature* **387**: 630-4 (1997)).
- Mutation to Glycine, Alanine, Valine, Leucine, Isoleucine, Methionine, Serine, Threonine, Asparagine or Glutamine. Some of these residues have small side chains, and all are uncharged at normal physiological pH. Mutations of one or several of residues 57-61 of human β₂m to any of this set of residues could cause loss of CD8 binding, either sterically or simply because the normal contacts between CD8 and β₂m are lost.
- Mutation to Proline. Proline induces structural restrictions in polypeptides and would therefore be predicted to alter the structure of the 57-61 loop of human β₂m, with expected loss of CD8 binding to the MHC complex. However, introduction of a Proline residue(s) may not be compatible with a functional β₂m structure, that is, folding of β₂m may be affected in such a way that the protein cannot form complexes with HLA heavy chains or have impaired ability to exchange into existing complexes on surface of cells.
- Mutation to Cysteine. Mutation to cysteine would create a 'free' SH group on the surface of β₂m being contacted by CD8. This may in itself inhibit CD8 contacts, but the residue could also be used to derivatise the β₂m polypeptide. For instance, a biotin group could be linked to β₂m, causing steric hindrance of CD8 binding if the mutated, derivatised β₂m polypeptide was exchanged into MHC complexes.

In addition to the mutations described above, β₂m may be mutated to increase its affinity for MHC so that its on-rate is greater and its off-rate is less than wild type β₂m, thereby resulting in greater occupancy of the mutant β₂m in the MHC. Mutations of this type are described in Schultz, *et al. Immunogenetics* **48**: 273-82 (1998).

Other possible mutations will be apparent to those skilled in the art. The MHC molecule of the present invention may exclude a Tyrosine₆₇ to Cysteine mutant.

A multimer of the present invention may be provided in substantially pure form. For example, it may be provided in a form which is substantially free of other proteins.

The skilled person will appreciate that a large number of conservative amino acid substitutions can be introduced in MHC and/or β₂m without causing any significant changes. Thus, for instance, proteins which include one or more additions, deletions, substitutions or the like are encompassed by the present invention. In addition, it may be possible to replace one amino acid with another of similar "type". For instance, replacing one hydrophobic amino acid with another. In the case of such homologues and derivatives, the degree of identity with a MHC as described above is less important than that the homologue or derivative should retain the ability to prevent T cell activation, preferably by causing apoptosis. However, suitably, homologues or derivatives having at least 60% identity are provided. Preferably, homologues or derivatives having at least 70% identity, more preferably at least 80% identity are provided. Most preferably, homologues or derivatives having at least 90% or even 95% identity are provided.

The percent identity of two amino acid sequences or of two nucleic acid sequences is determined by aligning the sequences for optimal comparison purposes (*e.g*., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared *(i.e.,* % identity = number of identical positions/total number of positions x 100).

The determination of percent identity between two sequences can be accomplished using a mathematical algorithm known to those of skill in the art. An example of a mathematical algorithm for comparing two sequences is the algorithm of Karlin and Altschul (1990) *Proc. Natl. Acad. Sci. USA* **87:**2264-2268**,** modified as in Karlin and Altschul (1993) *Proc. Natl. Acad. Sci. USA* **90**:5873-5877. The NBLAST and XBLAST programs of Altschul, *et al.* (1990) *J. Mol. Biol.* **215**:403-410 have incorporated such an algorithm. BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilised as described in *Altschul et al.* (1997) *Nucleic Acids Res.* **25**:3389-3402. Alternatively, PSI-Blast can be used to perform an iterated search which detects distant relationships between molecules *(Id.).* When utilising BLAST, Gapped BLAST, and PSI-Blast programs, the default parameters of the respective programs (e.g., XBLAST and NBLAST) can be used. See http://www.ncbi.nlm.nih.gov. Another example of a mathematical algorithm utilised for the comparison of sequences is the algorithm of Myers and Miller, CABIOS (1989). The ALIGN program (version 2.0) which is part of the CGC sequence alignment software package has incorporated such an algorithm. Other algorithms for sequence analysis known in the art include ADVANCE and ADAM as described in Torellis and Robotti (1994) *Comput. Appl. Biosci.,* **10** :3-5; and FASTA described in Pearson and Lipman (1988) *Proc. Natl. Acad. Sci.* **85:**2444-8. Within FASTA, ktup is a control option that sets the sensitivity and speed of the search.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient ability to inhibit the activation of a CD8⁺ T cell by a class I Major Histocompatibility Complex to be useful.

The multimers of the present invention can be provided alone, as a purified or isolated preparation. They may be provided as part of a mixture with one or more other proteins of the invention.

In yet further aspects, the present invention provides:
(a) the use of a multimer of the invention in allowing apoptosis to occur in a CD8⁺ T cell whilst inhibiting CD8⁺ T cell activation;
(b) the use of a multimer of the invention in the production of a medicament for allowing CD8⁺ T cell apoptosis to occur whilst inhibiting CD8⁺ T cell activation; and
(c) a method of allowing apoptosis in a CD8⁺ T cell to occur whilst inhibiting CD8⁺ T cell activation, the method comprising exposing the T cell to a multimer of the invention.

Gene cloning techniques may be used to provide a multimer of the invention in substantially pure form. These techniques are disclosed, for example, in J. Sambrook *et al Molecular Cloning* 2nd Edition, Cold Spring Harbor Laboratory Press (1989).

MHC molecules of the present invention are preferably expressed as soluble recombinant protein for extracellular addition. Alternatively, they may be expressed by transfection of a DNA construct, i.e. they may be administered using gene therapy. Transfection of DNA can be achieved both *in vitro* as well as *in vivo,* for example by using various types of recombinant viruses as vehicles for DNA transformation or by transfection techniques that use 'naked' DNA. For example, an organ to be transplanted may be incubated in a MHC molecule of the present invention to make it more difficult for the immune system of host to recognise and reject it.

Preferably in gene therapy, the MHC is administered such that it is expressed in the subject to be treated for example in the form of a recombinant DNA molecule comprising a polynucleotide(s) encoding the MHC chains operatively linked to a nucleic acid sequence which controls expression, such as in an expression vector.
Such a vector will thus include appropriate transcriptional control signals including a promoter region capable of expressing the coding sequence, said promoter being operable in the subject to be treated. Thus for human gene therapy, the promoter, which term includes not only the sequence necessary to direct RNA polymerase to the transcriptional start site, but also, if appropriate, other operating or controlling sequences including enhancers, is preferably a human promoter sequence from a human gene, or from a gene which is typically expressed in humans, such as the promoter from human cytomegalovirus (CMV). Among known eukaryotic promoters suitable in this regard are the CMV immediate early promoter, the HSV thymidine kinase promoter, the early and late SV40 promoters, the promoters of retroviral LTRs, such as those of the Rous sarcoma virus ("RSV"), and metallothionein promoters, such as the mouse metallothionein-I promoter.

A polynucleotide sequence and transcriptional control sequence may be provided cloned into a replicable plasmid vector, based on commercially available plasmids, such as pBR322, or may be constructed from available plasmids by routine application of well known, published procedures.

The vector may also include transcriptional control signals, situated 3' to the MHC encoding sequence(s), and also polyadenylation signals, recognisable in the subject to be treated, such as, for example, the corresponding sequences from viruses such as, for human treatment, the SV40 virus. Other transcriptional controlling sequences are well known in the art and may be used.

The expression vectors may also include selectable markers, such as for antibiotic resistance, which enable the vectors to be propagated.

Expression vectors capable *in situ* of synthesising MHC may be introduced directly by physical methods. Examples of these include topical application of the 'naked' nucleic acid vector in an appropriate vehicle for example in solution in a pharmaceutically acceptable excipient such as phosphate buffered saline (PBS). Other physical methods of administering the DNA directly to the recipient include ultrasound, electrical stimulation, electroporation and microseeding.

MHC encoding nucleic acid sequence(s) for use in the therapy of the invention may also be administered by means of delivery vectors. These include viral delivery vectors, such as adenovirus or retrovirus delivery vectors known in the art. Other non-viral delivery vectors include lipid delivery vectors, including liposome delivery vehicles, known in the art.

A MHC encoding nucleic acid sequence(s) may also be administered by means of transformed host cells. Such cells include cells harvested from the subject, into which the nucleic acid sequence is introduced by gene transfer methods known in the art, followed by growth of the transformed cells in culture and administration to the subject.

Expression constructs such as those described above may be used in a variety of ways in the therapy of the present invention. Thus, they may be directly administered to the subject, or they may be used to prepare MHC itself which can then be administered as is discussed in more detail below. The invention also relates to host cells which are genetically engineered with constructs which comprise MHC encoding polynucleotide(s), and to the uses of these vectors and cells in the therapeutic methods of the invention. These constructs may be used *per se* in the therapeutic methods of the invention or they may be used to prepare a MHC polypeptide for use in the therapeutic methods of the invention described in greater detail below.

The vector may be, for example, a plasmid vector, a single or double-stranded phage vector, a single or double-stranded RNA or DNA viral vector, depending upon whether the vector is to be administered directly (i.e. for *in situ* synthesis), or is to be used for synthesis of MHC. Starting plasmids disclosed herein are either commercially available, publicly available, or can be constructed from available plasmids by routine application of well known, published procedures. Many plasmids and other cloning and expression vectors that can be used in accordance with the present invention are well known and readily available to those of skill in the art.

Generally, vectors for expressing a polypeptide for use in the invention comprise cis-acting control regions effective for expression in a host operatively linked to the polynucleotide to be expressed. Appropriate trans-acting factors either are supplied by the host, supplied by a complementing vector or supplied by the vector itself upon introduction into the host.

In certain embodiments in this regard, the vectors provide for specific expression. For production of MHC, such specific expression may be inducible expression or expression only in certain types of cells or both inducible and cell-specific. Particularly preferred among inducible vectors are vectors that can be induced for expression by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

A great variety of expression vectors can be used to express MHC for use in the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, adeno-type viruses, fowl pox viruses, pseudorabies viruses and retroviruses, and vectors derived from combinations thereof, such as those derived from plasmid and bacteriophage genetic elements, such as cosmids and phagemids, all may be used for expression in accordance with this aspect of the present invention. Generally, any vector suitable to maintain, propagate or express polynucleotides to express a polypeptide in a host may be used for expression in this regard.

The appropriate DNA sequence may be inserted into the vector by any of a variety of well-known and routine techniques, such as, for example, those set forth in Sambrook et al., MOLECULAR CLONING, A LABORATORY MANUAL, 2nd Ed.; Cold Spring Harbor Laboratory Press, Cold Spring Harbor, New York (1989).

The nucleic acid sequence in the expression vector may be operatively linked to appropriate expression control sequence(s), including, for instance, a promoter to direct mRNA transcription. Representatives of such promoters include, but are not limited to, the phage lambda PL promoter, the *E. coli* lac, trp and tac promoters, for recombinant expression, and the SV40 early and late promoters and promoters of retroviral LTRs for *in situ* expression.

In general, expression constructs will contain sites for transcription initiation and termination, and, in the transcribed region, a ribosome binding site for translation.
The coding portion of the mature transcripts expressed by the constructs will include a translation initiating AUG at the beginning and a termination codon appropriately positioned at the end of the polypeptide to be translated.

In addition, the constructs may contain control regions that regulate as well as engender expression. Generally, in accordance with many commonly-practised procedures, such regions will operate by controlling transcription, such as transcription factors, repressor binding sites and termination, among others.

Vectors for propagation and expression generally will include selectable markers and amplification regions, such as, for example, those set forth in Sambrook et al., MOLECULAR CLONING, A LABORATORY MANUAL, 2nd Ed.; Cold Spring Harbor Laboratory Press, Cold Spring Harbor, New York (1989).

Representative examples of appropriate hosts for recombinant expression of MHC include bacterial cells, such as *streptococci, staphylococci, E. coli, streptomyces* and *Bacillus subtilis* cells; fungal cells, such as yeast cells and *Aspergillus* cells; insect cells such as *Drosophila* S2 and *Spodoptera* Sf9 cells; animal or human cells such as CHO, COS, HeLa, C127, 3T3, BHK, 293 and Bowes melanoma cells; and plant cells.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRITS available from Pharmacia, and pBR322 (ATCC 37017). Among preferred eukaryotic vectors are pWLNEO, pSV2CAT, pOG44, pXT1 and pSG available from Stratagene; and pSVK3, pBPV, pMSG and pSVL available from Pharmacia. These vectors which can be used both for recombinant expression and for *in situ* expression are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide for use in therapy in a host may be used.

Examples of vectors include expression vectors in which the MHC cDNA sequence is inserted in a plasmid whereby gene expression is driven from the human immediate early cytomegalovirus enhancer-promoter (Foecking and Hofstetter, *Cell*, **45**, 101-105, 1986). Such expression plasmids may contain SV40 RNA processing signals such as polyadenylation and termination signals. Expression constructs which use the CMV promoter and that are commercially available are pCDM8, pcDNA1 and derivatives, pcDNA3 and derivatives (Invitrogen). Other expression vectors available which may be used are pSVK3 and pSVL which contain the SV40 promoter and mRNA splice site and polyadenylation signals from SV40 (pSVK3) and SV40 VP1 processing signals (pSVL; vectors from Pharmacia).

Promoter regions can be selected from any desired gene using vectors that contain a reporter transcription unit lacking a promoter region, such as a chloramphenicol acetyl transferase ("CAT") transcription unit, downstream of restriction site or sites for introducing a candidate promoter fragment; i.e., a fragment that may contain a promoter. As is well known, introduction into the vector of a promoter-containing fragment at the restriction site upstream of the cat gene engenders production of CAT activity, which can be detected by standard CAT assays. Vectors suitable to this end are well known and readily available, such as pKK232-8 and pCM7. Promoters for expression of polynucleotides for use in the therapy of the present invention include not only well known and readily available promoters, but also promoters that readily may be obtained by the foregoing technique, using a reporter gene; *for in situ* expression, such a promoter should be recognised in the subject to be treated.

Among known prokaryotic promoters suitable for expression of polynucleotides and polypeptides in accordance with the therapy of the present invention are the *E*. *coli* lacI and lacZ and promoters, the T3 and T7 promoters, the gpt promoter, the lambda PR, PL promoters and the trp promoter.

Recombinant expression vectors will include, for example, origins of replication, a promoter preferably derived from a highly-expressed gene to direct transcription of a downstream structural sequence, and a selectable marker to permit isolation of vector containing cells after exposure to the vector.

Polynucleotides for use in therapy generally will be inserted into the vector using I standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed.

Generally, there will be no other open reading frames that begin with an initiation codon, usually AUG, and lie between the ribosome binding site and the initiation codon. Also, generally, there will be a translation stop codon at the end of the polypeptide and there will be a polyadenylation signal in constructs for use in eukaryotic hosts. Transcription termination signal appropriately disposed at the 3' end of the transcribed region may also be included in the polynucleotide construct.

For secretion of the translated protein into the lumen of the endoplasmic reticulum, into the periplasmic space or into the extracellular environment, appropriate secretion signals may be incorporated into the expressed polypeptide when recombinantly synthesised. These signals may be endogenous to the polypeptide or they may be heterologous signals.

The polypeptide may be expressed in a modified form, such as a fusion protein, and may include not only secretion signals but also additional heterologous functional regions. Thus, for instance, a region of additional amino acids, particularly charged amino acids, may be added to the N- or C-terminus of the polypeptide to improve stability and persistence in the host cell, during purification or during subsequent handling and storage. Also, a region may be added to the polypeptide to facilitate purification. Such regions may be removed prior to final preparation of the polypeptide. The addition of peptide moieties to polypeptides to engender secretion or excretion, to improve stability or to facilitate purification, among others, are familiar and routine techniques in the art. A preferred fusion protein comprises a heterologous region from immunolglobulin that is useful to solubilise or purify polypeptides. Cells typically then are harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Mammalian expression vectors may comprise an origin of replication, a suitable promoter and enhancer, and also any necessary ribosome binding sites, polyadenylation regions, splice donor and acceptor sites, transcriptional termination sequences, and 5' flanking non-transcribed sequences that are necessary for expression.

For preparing polypeptides for use in the invention, genetically engineered host cells may be used. Introduction of a polynucleotide into the host cell can be affected by calcium phosphate transfection, DEAE-dextran mediated transfection, transvection, microinjection, cationic lipid-mediated transfection, electroporation, transduction, scrape loading, ballistic introduction, infection or other methods. Such methods are described in many standard laboratory manuals, such as Davis et al., BASIC METHODS IN MOLECULAR BIOLOGY, (1986) and Sambrook *et al.,* MOLECULAR CLONING: A LABORATORY MANUAL, 2nd Ed., Cold Spring Harbor Laboratory Press, Cold Spring Harbor, N.Y (1989).

Mature proteins can be expressed in host cells including mammalian cells such as CHO cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook et al., MOLECULAR CLONING: A LABORATORY MANUAL, 2nd Ed., Cold Spring Harbor Laboratory Press, Cold Spring Harbor, N.Y. (1989).

The polypeptide can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding protein may be employed to regenerate active conformation when the polypeptide is denatured during isolation and or purification.

Binding of CD8 to MHC/peptide complexes can be conveniently detected by surface plasmon resonance studies, for instance on the Biacore2000 or Biacore3000 systems *(Garcia, et al. Nature* **384:** 577-81 Issn: 0028-0836 (1996); Wyer, *et al. Immunity* **10:** 219-225 (1999)). Thus, CD8 binding to sensor cells with immobilised MHC/peptide complexes could be measured, one cell serving as control for other sensor cells on which the MHC/peptide complex includes modified β₂m. Such analysis will provide a biophysical measurement of the degree to which the MHC molecules are capable of inhibiting CD8 binding.

The inhibitory effects of MHC molecules of the invention can be tested in *in vitro* CTL assays in order to assess their effect on T cell responses, and especially if they cause apoptosis of T cells. These studies can be extended to *in vivo* analysis of the effects of the MHC molecules, by testing these in relevant animal disease models.

Medicaments in accordance with the invention will usually be supplied as part of a sterile, pharmaceutical composition which will normally include a pharmaceutically acceptable carrier. This pharmaceutical composition may be in any suitable form, (depending upon the desired method of administering it to a patient).

It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions)

Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc.

For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in *Pharmaceutical Research,* **3(6**):318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the substances of the present invention can vary between wide limits, depending upon the disease or disorder to be treated, the age and condition of the individual to be treated, etc. and a physician will ultimately determine appropriate dosages to be used. The dosage may be repeated as often as appropriate. If side effects develop the amount and/or frequency of the dosage can be reduced, in accordance with normal clinical practice.

MHC multimers of the present application also find use in isolating and/or selecting T cells. For example, multimers comprising three MHC molecules of the invention and a detectable label are brought into contact with a sample comprising T cells. The multimers bind to the T cells allowing them to be isolated/selected. Because the multimers inhibit CD8 on the T cell binding to the tetramers, T cells are selected by virtue of their interaction with the MHC molecules and/or peptides of the multimer and the contribution of CD8 to binding is minimised. T cells can be selected/isolated according to the MHC/peptide complexes used in the multimers. Thus, in a still further aspect, the present invention provides a method for selecting/isolating T cells comprising bringing a sample containing T cells into contact with a multimer of the invention and selecting/isolating T cells which bind to the multimer.

Preferred features of each aspect of the invention are as for each of the other aspects *mutatis mutandis.* The prior art documents mentioned herein are incorporated to the fullest extent permitted by law.

The invention will now be described further in the following non-limiting examples. Reference is made to the accompanying drawings in which:
Figure 1 a shows the amino acid sequence of the processed human β₂m polypeptide and the DNA sequence encoding it, and Figures 1b-m show the modifications that need to be made to this DNA sequence to introduce mutations in accordance with the invention, and the amino acid sequences of the relevant regions of resulting β₂m proteins;
Figure 2 shows a Coomassie-stained SDS-PAGE gel of the *Escherichia coli* strain BL21-DE3 (pLysS) expressing human β₂m;
Figure 3 shows a Coomassie-stained SDS-PAGE gel with purified inclusion bodies containing denatured human β₂m;
Figure 4 shows a gel filtration trace of refolded human β₂m, the correctly folded protein eluting after 220 ml;
Figure 5 shows a SDS-PAGE gel with size markers in Lane 1 and refolded β₂m in Lane 2;
Figure 6 shows schematically a procedure for determining the ability of β₂m mutant proteins to exchange and to inhibit cytotoxic lymphocyte activation;
Figures 7a-e show BIAcore responses showing the ability of FLU-HLA-A2/β₂m complexes containing mutated β₂m to bind soluble CD8αα compared to that of complexes containing wild type β₂m;
Figures 8a-e show BIAcore responses showing the ability of HLA-A2/β₂m complexes containing mutated β₂m to bind TCR compared to that of complexes containing wild type β₂m;
Figures 9a and 9b show BIAcore responses showing the ability of HLA-A2/β₂m complexes containing mutated β₂m to bind CD8 and TCR respectively after storage for 24 hours, compared to that of complexes containing wild type β₂m;
Figure 10 is a plasmid map of the plasmid pIRES;
Figure 11 shows the coding sequence of HLA-A*02011, together with primer sequences used in the present invention;
Figure 12 shows the coding sequence for full length wild type β2m;
Figure 13 is a graph illustrating the inhibition of HLA-A2 restricted CTL clone 5D8 by mutant HLA/β2m complexes;
Figure 14 is a graph illustrating the inhibition of HLA-A2 restricted parvovirus B 19 specific CTL line by mutant HLA/β2m complexes;
Figure 15 shows schematically a procedure for investigating human HLA multimer containing mβ₂m inhibition of CTL priming *in vivo;* and
Figure 16 shows the DNA sequence of a plasmid, pBJ196, used for expression of peptide-β₂microglobulin fusions in *E.coli.*

### Examples

### Example 1 - K₅₈ → E mutation of human β₂m

This example describes the construction of the DNA expression plasmid pEX052 which codes for the β₂m in which Lysine₅₈ is substituted for Glutamate. The DNA sequences of the primers used are shown highlighted in Figure 1b (K58 → E); the sequence on top corresponds to the sense strand of β₂m, the sequence on the bottom corresponds to the non-sense strand of β₂m. Mutated bases are indicated in small letters and the amino acid sequence is indicated below the DNA sequences.

A DNA plasmid, pBJ192, which encodes human β₂m in which the signal peptide is substituted for a single Methionine residue in order to allow initiation of translation when expressed in bacteria, was generated as follows. A PCR reaction was performed on cDNA generated from a human B cell line with the primers 5'- GGG GGG CAT ATG ATt CAa aGa ACT CCA Aaa ATT CAG GTT TAC TCA CGT CAT CC -3' (forward primer) and 5'- GGG GGA AGC TTA CAT GTC TCG ATC CCA CTT AAC TAT - 3' (backward primer). Bases shown in small letters indicate mutations in relation to the human sequence. These do not alter the sequence of the polypeptide that is encoded, in relation to the human sequence, but were introduced in order to increase expression of the β₂m protein in *E. coli.* The PCR product was cloned into pGMT7 (Studier, *et al. Methods Enzymol* 185: 60-89 Issn: 0076-6879 (1990)) at the restriction sites for NdeI and HindIII.

100 ng of plasmid pBJ192 was mixed with 5 µl 10 mM dNTP, 25 µl 10xPfu-buffer (Stratagene), 10 units Pfu polymerase (Stratagene) and the final volume was adjusted to 240 µl with H₂O. 48 µl of this mix was supplemented with primers diluted to give a final concentration of 0.2 µM in 50 µl final reaction volume. After an initial denaturation step of 30 seconds at 95° the reaction mixture was subjected to 15 rounds of denaturation (95°C, 30 sec.), annealing (55°C, 60 sec.), and elongation (73°C, 8 min.) in a Hybaid PCR express PCR machine. The product was then digested for 5 hours at 37°C with 10 units of DpnI restriction enzyme (New England Biolabs). 10 µl of the digested reaction was transformed into XL1-Blue bacteria and grown for 18 hours at 37°C. A single colony was picked and grown over night in 5 ml TYP + ampicillin (16 g/l Bacto-Tryptone, 16 g/l Yeast Extract, 5 g/l NaCl, 2.5 g/l K₂HPO₄, 100 mg/l Ampicillin). Plasmid DNA was purified on a Qiagen mini-prep column according to the manufacturer's instructions and the sequence was verified by automated sequencing at the sequencing facility of Department of Biochemistry, Oxford University.

### Example 2 - K₅₈ → S mutation of human β₂m

This example describes the construction of the DNA expression plasmid pEX053 which codes for the β₂m in which Lysine₅₈ is substituted for Serine. The DNA sequences of the primers used are shown in Figure 1c (K58 → S). Mutation of DNA plasmid pBJ192 was carried out according to the same protocol as described in Example 1 and the sequence was verified by automated sequencing at the sequencing facility of Department of Biochemistry, Oxford University.

### Example 3 - K₅₈ → R mutation of human β₂m

This example describes the construction of the DNA expression plasmid pEX051 which codes for the β₂m in which Lysine₅₈ is substituted for Arginine. The DNA sequences of the primers used are shown in Figure 1d (K58 → R). Mutation of DNA plasmid pEX052 was carried out according to the same protocol as described in Example 1 except that 20 PCR rounds were carried out and the elongation time was increased to 10 minutes. The sequence was verified by automated sequencing at the sequencing facility of Department of Biochemistry, Oxford University.

### Example 4 - K₅₈ → Y mutation of human β₂m

This example describes the construction of the DNA expression plasmid pEX054 which codes for the β₂m in which Lysine₅₈ is substituted for Tyrosine. The DNA sequences of the primers used are shown in Figure 1e (K58 → Y). Mutation of DNA plasmid pEX052 was carried out according to the same protocol as described in Example 3 and the sequence was verified by automated sequencing at the sequencing facility of Department of Biochemistry, Oxford University.

### Example 5 - K₅₈ → C mutation of human β₂m

This example describes the construction of the DNA expression plasmid pEX055 which codes for the β₂m in which Lysine₅₈ is substituted for Cysteine. The DNA sequences of the primers used are shown in Figure 1f (K58 → C). Mutation of DNA plasmid pEX052 was carried out according to the same protocol as described in Example 3 and the sequence is verified by automated sequencing at the sequencing facility of Department of Biochemistry, Oxford University.

### Example 6 - K₅₈ → SES mutation of human β₂m

This example describes the construction of the DNA expression plasmid pEX056 which codes for the β₂m in which Lysine₅₈ is substituted for Serine-Glutamate-Serine. The DNA sequences of the primers used are shown in Figure 1g (K58 → SES). Mutation of DNA plasmid pEX052 was carried out according to the same protocol as described in Example 3 and the sequence was verified by automated sequencing at the sequencing facility of Department of Biochemistry, Oxford University.

### Example 7 - K₅₈ → W mutation of human β₂m

This example describes the construction of the DNA expression plasmid pEX061 which codes for the β₂m in which Lysine₅₈ is substituted for Tryptophan. The DNA sequences of the primers used are shown in Figure 1h (K58 → W). Mutation of DNA plasmid pEX051 was carried out according to the following protocol. 10 ng of template plasmid (pEX051) was mixed with 1.25 µl 10 mM dNTP, 5 µl 10xPfu-buffer (Stratagene), 2.5 units Pfu polymerase (Stratagene) and the final volume was adjusted to 50 µl with H₂O. 48 µl of this mix was supplemented with primers diluted to give a final concentration of 0.2 µM in 50 µl final reaction volume. After an initial denaturation step of 2 minutes at 95°C the reaction mixture was subjected to 18 rounds of denaturation (95°C, 30 sec.), annealing (55°C, 60 sec.), and elongation (68°C, 10 min.) in a Hybaid PCR express PCR machine. The product was then digested for 1 hour at 37°C with 20 units of DpnI restriction enzyme (New England Biolabs). 10 µl of the digested reaction was transformed into XL1-Blue bacteria and grown for 18 hours at 37°C. A single colony was picked and grown over night in 5 ml TYP + ampicillin (16 g/l Bacto-Tryptone, 16 g/l Yeast Extract, 5 g/l NaCl, 2.5 g/l K₂HPO₄, 100 mg/l Ampicillin). Plasmid DNA was purified on a Qiagen Spin mini-prep column according to the manufacturer's instructions and the sequence was verified by automated sequencing at the sequencing facility of Department of Biochemistry, Oxford University.

### Example 8 - K₅₈ → GRG mutation of human β₂m

This example describes the construction of the DNA expression plasmid pEX062 which codes for the β₂m in which Lysine₅₈ is substituted for the tri-peptide Glycine-Arginine-Glycine. The DNA sequences of the primers used are shown in Figure 1 i (K58 → GRG). Mutation of DNA plasmid pEX056 was carried out according to the same protocol as described in Example 7 and the sequence was verified by automated sequencing at the sequencing facility of Department of Biochemistry, Oxford University.

### Example 9 - D₅₉ → GEG mutation of human β₂m

This example describes the construction of the DNA expression plasmid pEX063 which codes for the β₂m in which Aspartate₅₉ is substituted for the tri-peptide Glycine-Glutamate-Glycine. The DNA sequences of the primers used are shown in Figure 1j (D59 → GEG). Mutation of DNA plasmid pEX050 was carried out according to the same protocol as described in Example 7 and the sequence was verified by automated sequencing at the sequencing facility of Department of Biochemistry, Oxford University.

### Example 10 - W₆₀ → G mutation of human β₂m

This example describes the construction of the DNA expression plasmid pEX064 which codes for the β₂m in which Tryptophan₆₀ is substituted for Glycine. The DNA sequences of the primers used are shown in Figure 1k (W60 → Y). Mutation of DNA plasmid pEX050 was carried out according to the same protocol as described in Example 7 and the sequence was verified by automated sequencing at the sequencing facility of Department of Biochemistry, Oxford University.

### Example 11 - K₅₈ W₆₀ → RG mutation of human β₂m

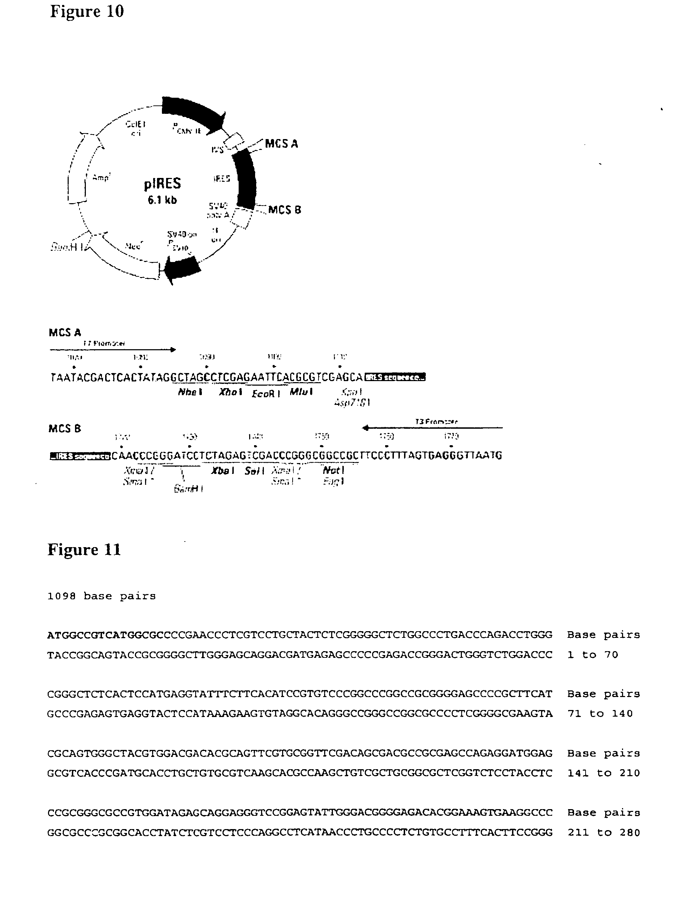

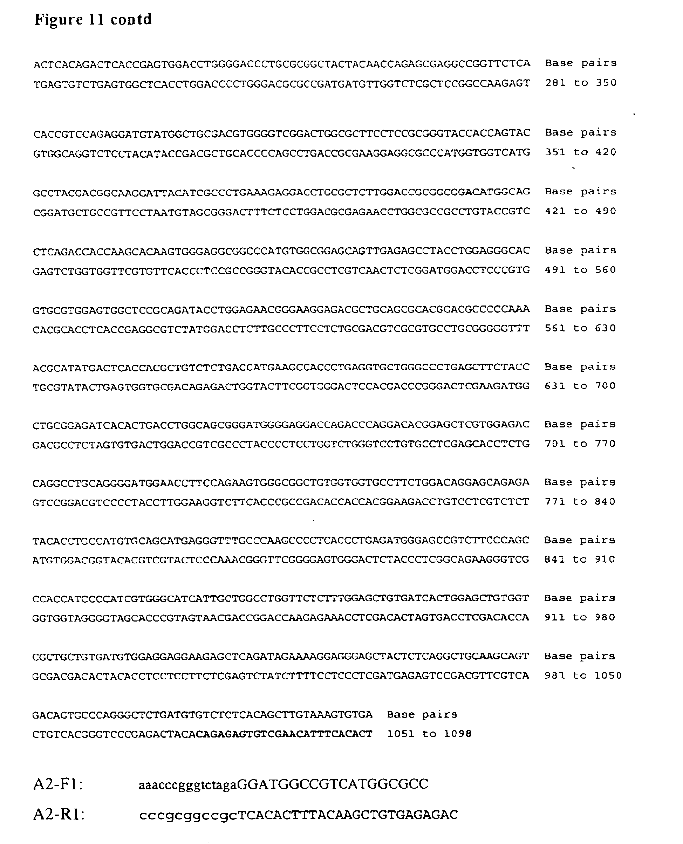

This example describes the construction of the DNA expression plasmid pEX065 which codes for the β₂m in which Lysine₅₈ and Tryptophan ₆₀ are substituted for Arginine and Glycine respectively. The DNA sequences of the primers used are shown in Figure 11 (K58W60 → RG). Mutation of DNA plasmid pEX051 was carried out according to the same protocol as described in Example 7 and the sequence was verified by automated sequencing at the sequencing facility of Department of Biochemistry, Oxford University.

### Example 12 - K₅₈ → D mutation of human β₂m

This example describes the construction of the DNA expression plasmid pEX090 which codes for the β₂m in which Lysine₅₈ is substituted for Aspartate. The DNA sequences of the primers used are shown in Figure 1 m (K58 → D). Mutation of DNA plasmid pBJ192 was carried out according to the same protocol as described in Example 1 and the sequence was verified by automated sequencing at the sequencing facility of Department of Biochemistry, Oxford University.

### Example 13 - Expression, refolding and purification of human β₂m and β₂m mutants

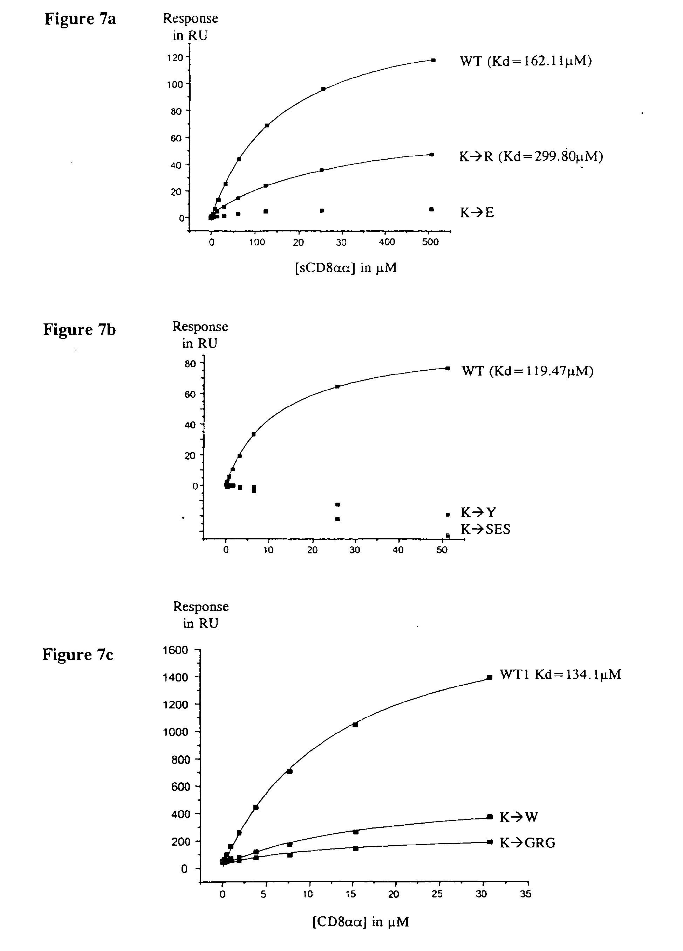

β₂m protein was expressed from the DNA vector pBJ 192, and from mutated derivatives of pBJ192, e.g. pEX051-56, pEX061-65 and pEX090, in the *Eschericia coli* strain BL21-DE3 pLysS (Novagen). pBJ192 contains the β₂m gene under the control of the strongly inducible T7 promoter in the vector pGMT7 (Studier, *et al. Methods Enzymol* **185***:* 60-89 Issn: 0076-6879 (1990)). The BL21 cells transformed with one of the β₂m-expressing vectors were plated on LB/agar/100 mg/ml ampicillin plates made according to a standard recipe. Transformants were then grown in TYP medium with Ampicillin (16 g/l Bacto-Tryptone, 16 g/l Yeast Extract, 5 g/l NaCl, 2.5 g/l K₂HPO₄, 100 mg/l Ampicillin) to an OD₆₀₀~ 0.4. For large-scale expression, 11 volumes of TYP media were prepared in 2 1 conical flasks and were covered with four layers of aluminium foil and were autoclaved. Cell densities were measured using optical density at 600 nm wavelength (OD600) on a Beckman DU530 spectrophotometer. Sterile TYP media was used as a blank. Figure 2 shows a Coomassie-stained SDS-PAGE gel of the *Escherichia coli* strain BL21-DE3 (pLysS) expressing human β₂m. Lane 1 contains a bacterial extract before induction of T7 transcription, lane 2 shows an extract from the same bacterial culture, but after 3 hours of induction of T7 transcription. Prior to induction, very little β₂m was produced by the bacteria, but after induction with 0.5 mM Iso-phenyl thio-galactoside (IPTG, from Melford), large amounts of β₂m were produced and deposited in inclusion bodies. Inclusion bodies were purified as described (Gao, *et al, Prot. Sci.***7***:* 1245-49 (1998)). Cells were lysed in 'Lysis Buffer' (10 mM EDTA (from 0.5 M stock pH 8.0), 2 mM DTT (from 1 M stock in 10 mM sodium acetate pH 5.2, stored at -20°C), 10 mM Tris pH 8.1 (from 2 M stock pH 8.1), 150 mM NaCl (from 4 M stock), 200 µg/ml lysozyme (from 20 mg/ml stock stored at -20°C), 10% glycerol (from fluid), 2500 units of DNAase I and 10mM MgCl₂ using a 50 ml Dounce homogeniser DNase I and lysozyme were from Sigma). Sonication, in lysis buffer, to break open the cells was performed using a 12mM probe sonicator (Milsonix XL2020). The probe was tuned according to the manufacturers instructions. The resulting suspension was then diluted 1:1 in 'Triton Buffer' (0.5% (w/v) Triton X-100 (from fluid), 50 mM Tris pH 8.1 (from 2 M stock), 100 mM NaCl (from 4 M stock), 0.1% sodium azide (from solid), 10 mM EDTA (from 0.5 M stock pH 8.0), 2 mM DTT (from 1 M stock in 10 mM sodium acetate pH 5.2, stored at -20°C) and left overnight. The inclusion bodies were separated from cell debris by centrifugation in a Beckman J2-21 centrifuge equipped with a JA-20 rotor as described *(Gao, et al, Prot. Sci.*7*:* 1245-49 (1998)) and stored at -20°C. Inclusion bodies were then thawed and resuspended in 'Resuspension Buffer' (50 mM Tris pH 8.1 (from 2 M stock), 100 mM NaCl (from 4 M stock), 10 mM EDTA (from 0.5 M stock pH 8.0), 2 mM DTT (from 1 M stock in 10 mM sodium acetate pH 5.2, stored at -20°C)), and denatured in 6M Guanidine and 10mM DTT buffered with Tris-HCl pH 8.1 (all chemicals from Sigma). Figure 3 shows a Coomassie-stained SDS-PAGE gel with purified inclusion bodies containing denatured human β₂m. β₂m is then refolded *in vitro* in the presence of 0.4M L-Arginine and purified by gel filtration chromotography, for instance on a Pharmacia Superdex 75 column. Figure 4 shows a gel filtration trace of refolded human β₂m, the correctly folded protein eluting after 220 ml. β₂m protein typically expresses at a level > 100 mg/l medium and refolds at an efficiency of ~36% giving high yields of correctly refolded soluble protein. It elutes from the Superdex 75 PG (preparation grade 26/60) column (Pharmacia) at an elution volume of 220 ml. It was judged, by Coomassie-stained SDS-PAGE, to be >95% pure after the single purification step.

Figure 5 shows a SDS-PAGE gel with size markers in Lane 1 and refolded β₂m in Lane 2.

### Example 14 - Testing of CD8 binding to HLA complex incorporating β₂m mutant proteins

β₂m mutant proteins were prepared according to Example 13, using the plasmids described in Examples 1-12. After the purification of denatured β₂m mutant protein, refolding was carried out in the presence of peptide and denatured HLA heavy chain *(Gao, et al, Prot. Sci.***7*****:*** 1245-49 (1998)) containing a tag sequence that can be enzymatically biotinylated (Schatz, *Biotechnology N Y* **11**: 1138-43 (1993); Altman, *et al Science* **274:** 94-6 (1996); Wyer, *et al. Immunity* **10**: 219-225 (1999)). The complex was then biotinylated using the enzyme BirA (O'Callaghan, *et al. Anal Biochem* **266**(1): 9-15 (1999)) to produce mutant β₂m- HLA-A2 - peptide complexes which were biotinylated towards the C-terminus of the HLA-A2 heavy chain. These protein complexes were immobilised on a streptavidin-modified BIAcore chip sensor cell in a BIAcore 3000 machine. The binding of soluble CD8αα were monitored by surface plasmon resonance when the CD8 protein was caused to flow over the sensor cell at a concentration in the range of 10-20 mg/ml.

Determination of the effects on CD8 binding of mutations introduced in the β₂m protein was accomplished with an experimental set up as illustrated in Figure 6. The HLA (or MHC) complex involving non-mutated β₂m protein was immobilised in one sensor cell, the HLA complexes involving each mutated β₂m protein were immobilised in each other sensor cell. To test that all complexes were correctly folded, soluble TCR, specific for the HLA/peptide complex used, was passed through all the sensor cells used (Wyer, *et al. Immunity* **10:** 219-225 (1999)). Detection of the antigen specific interaction is an extremely strong indication that folding of the HLA complex is correct. Next, soluble CD8 protein was passed over all of the sensor cells and the levels of binding in these were compared. If binding of soluble CD8 in a sensor cell was absent, or significantly reduced, then it was concluded that the mutant β₂m protein incorporated into this complex had affected CD8's ability to bind the complex.

The following β₂m mutants were prepared.

| Mutation | Plasmid |
|---|---|
| 58K → R | pEX 051 |
| 58K → E | pEX 052 |
| 58K → S | pEX 053 |
| 58K → Y | pEX 054 |
| 58K → C | pEX 055 |
| 58K → SES | pEX 056 |
| 58K → W | pEX061 |
| 58K → GRG | pEX062 |
| 59D → GEG | pEX063 |
| 60W → G | pEX064 |
| 59K/60W→ R/G | pEX065 |
| 58K → D | pEX090 |

The ability of HLA-A2/β₂m complexes containing the above mutations to bind soluble CD8αα was compared to that of complexes containing WT β₂m, as described above.

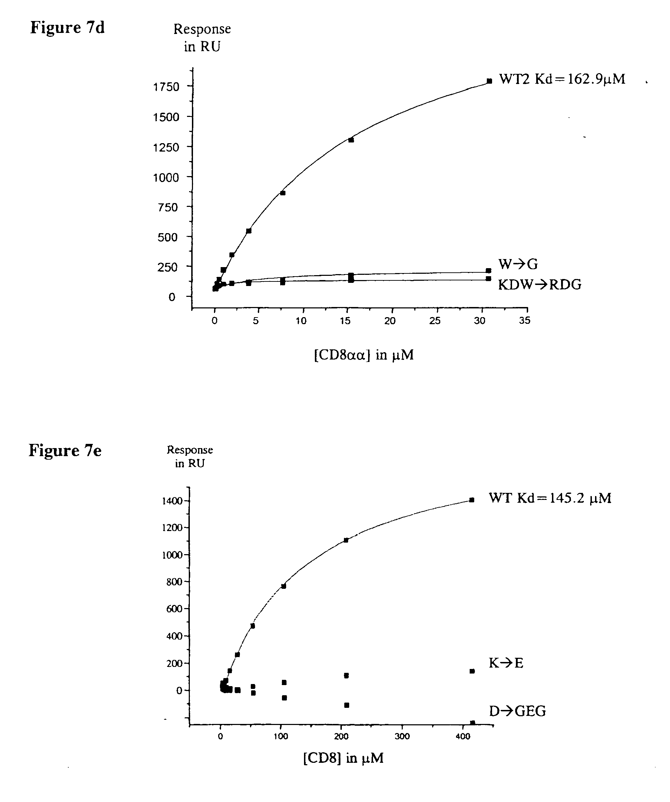

The results of studies to determine the binding of HLA-A2/mutant β₂m complexes to bind soluble CD8αα are shown in Figures 7a-e.

The concentration in µM is calculated from the measured absorbance of the sCD8αα stock solution (Conc = Absx10⁶/(extinct co x Mw)). The response is the difference between the response obtained for one cell containing a complex and the reference cell and is indicated in Response Units. The concentration of sCD8αα in µM was plotted (x) against the response in RU (y) and the points were fitted in the equation: Response = n[CD8]/([CD8] + Kd).

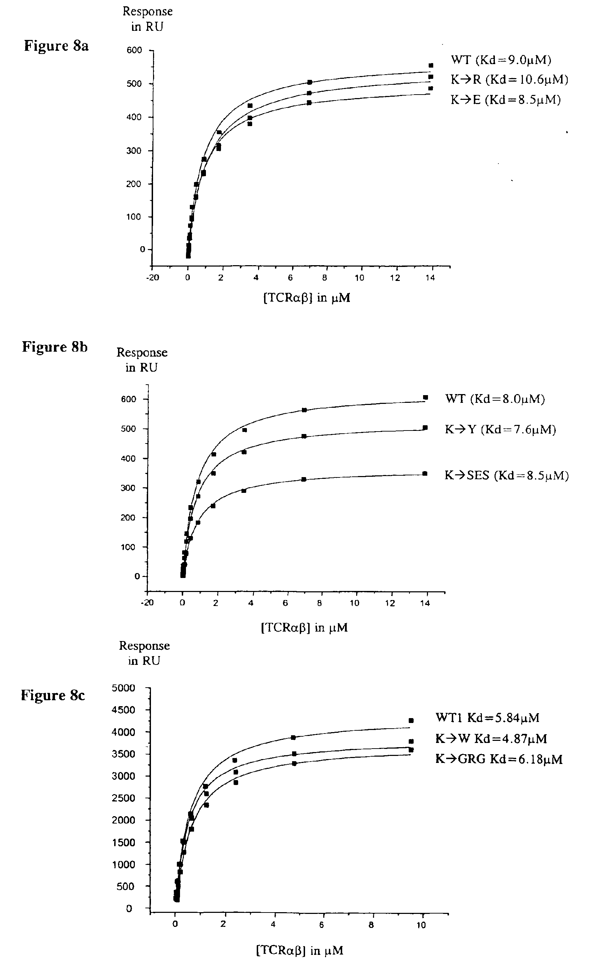

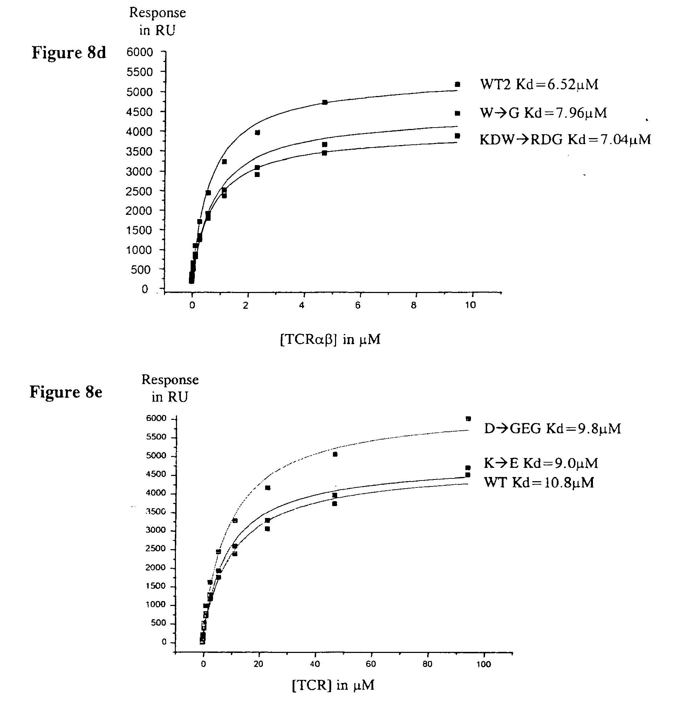

The ability of the HLA/mutant β₂m complexes to be conveniently refolded and to bind to the appropriate TCR, were also investigated. The results are shown in Figures 8a-e. The concentration of TCR µM was plotted (x) against the response in RU (y) and the points were fitted in the equation Response = n[TCR]/([TCR] + Kd).

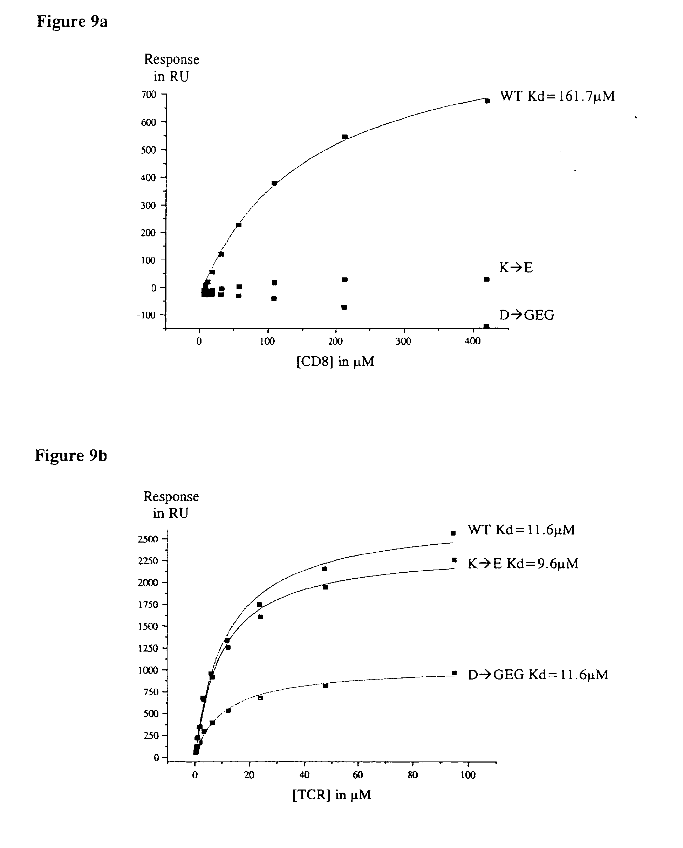

The stability of the HLA-A2-mutant β₂m complexes was also investigated for the 58K→ E and 59D → GEG mutants by re-assessing their ability to bind CD8 and TCR after storage at room temperature on a BIA core chip for 24 hours. The results are shown in Figures 9a and 9b respectively. The 24 hour stability assessment demonstrated no change in the CD8 or TCR binding response for the 58K→E mutant. However the TCR binding of the 59D→ GEG mutant demonstrated a drop in total binding response with a practically unchanged Kd.

The results obtained (see Figures 7a-e) demonstrate that the HLA-A2 β₂m substitution mutations cause a decrease in affinity for soluble CD8αα. Almost total inhibition of CD8 binding was caused by the following mutations:
K58→SES, K58→E, K58→Y, W60→G, K58/W60→R/G, K58→W, D59→GEG and K58→GRG.

Partial inhibition of CD8 binding was caused by the following mutations, in order of decreasing inhibition:
K58→R > K58→C > K58→S.

The recovery on refolding of the mutants was also assessed (see Figures 8a-e) and the mutants are listed in order of decreasing recovery:
K58→R = K58→E > K58→Y = K58→W > K58→C = K58→S > K58→SES = D59→GEG = K58→GRG > W60→G = K58/W60→R/G

The ability of the mutants to bind TCR was also assessed (see Figures 8a-e) and the mutants tested are listed in order of decreasing total binding response:
K58 → R = D59→GEG = K58→E > K58→Y = K58→W > K58→SES = K58→GRG = W60→G = K58/W60→R/G (K58→C & K58→S not tested)

Among the mutants that totally inhibit CD8 binding, only K58→E refolds as well as WT β2m and is fully functional. The 24 hour storage tests indicate that the K58→E mutant is stable over this time period whereas the D59→GEG mutant undergoes partial degradation.

### Example 15 - Compounds which may be attached to Cysteine in the β₂m K₅₈ → C mutant and linkers which may effect the attachment.

Mutation of Lysine₅₈ in β₂m to a Cysteine residue allows the unpaired SH group of the Cysteine to be used to link other compounds to the mutated β₂m protein. Such other compounds are used sterically to hinder CD8 binding to HLA (or MHC) complexes of which the mutated and derivatised β₂m forms a part. This strategy is similar to those described above where mutation to residues with larger side chains are used to prevent or inhibit CD8 binding. The difference with using derivatisation of the SH group of Cysteine at position 58 is that relatively larger compounds can be incorporated into the protein. This can cause a more efficient steric hindrance than can be achieved with non-derivatised amino acid mutations. However, this involves a further step in the preparation and may reduce the ability of the protein to exchange into HLA/peptide complexes.

Compounds that can be used to derivatise β₂m K₅₈ → C include amino acids, peptides, Biotin, and DNA, all of which would be expected to severely affect CD8's ability to slot into its binding site on HLA (or MHC) complexes.

Derivatisation is performed with a suitable linker molecule, such as Maleimide, Iodoacetamide, 3,-(pyridyldithio)propionate and 3,-(pyridyldithio)propionamide, all of which are commercially available from chemical reagent companies (Sigma, Aldrich, Pierce). These linker compounds can be used to attach an almost limitless variety of groups to a Cysteine residue, either by producing a linker-derivatised molecule (e.g. 3-(N-Maleimidopropionyl) Biocytin^{™} available from Sigma) or by using a heterobifunctional molecule such as e-Maleimidocaproic acid N-hydroxysuccinimide ester (Sigma) to conjugate e.g. a protein.

### Example 16 - HLA/mutant β2m mammalian expression vector for immunosuppressive therapy

There are a number of expression systems, known to those skilled in the art, which can be appropriate for the therapeutic delivery of the proteins of the present invention. DNA can be delivered by a number of different established routes using viral vectors, injection of naked DNA into the recipient tissue in a formulation optimised for cellular uptake, injection using accelerated DNA coated particles, or any technology designed for *in vivo* gene delivery.

HLA/mutant β2m complexes can be administered against CTL based immune disorders using modified adeno-associated virus. This is a vector which can be used for the therapeutic delivery of DNA, and is capable of infecting a wide rage of tissue types and therefore could be used for a variety of applications.

Tissue specificity of expression can be controlled by using tissue specific promoter and enhancer sequences in conjunction with a general mode of delivery. Tissue specificity can also be obtained by using viral vectors which only infect certain cell or tissue types in conjunction with using general regulatory elements controlling the expression of the genes. The vector and regulatory sequence combination of choice will depend on the nature of the disease being treated.

The human creatine kinase (CKM) promotor is an example of a tissue-specific regulatory sequence capable of targeted DNA expression in muscle tissue. The human cytomegalovirus (CMV) promoter is an example of a regulatory sequence capable of inducing DNA expression in a broad range of tissues.

### Example 17 - Production of HLA-β2m mammalian expression vector for in vitro T cell assays

This example details the protocol used to produce an appropriate bicistronic vector for the production of wild type (WT) and HLA-mutant β₂m expression vectors, which can be used to induce expression of these proteins for *in-vitro* T cell assays. 10⁷ T2 cells (Payne *et* al, *Immunogenetics* (1990) **31**(3):169-78) cells were harvested and lysed in 1 ml of TRI reagent from Sigma and total RNA was harvested according to instructions provided by the manufacturer. 10 µg total RNA, 0.01 OD₂₆₀ of d(pT)₁₂₋₁₈ primer, 10 µl of 10xRT buffer and 5 µl Omniscript Reverse Transcriptase (Qiagen Omniscript RT-kit Cat. No. 20511 lot EGQ002) was used for cDNA synthesis in a total volume of 100 µl according to instructions provided by the manufacturer. PCR was performed on 0.5 µl T2 cDNA in a total volume 40 µl using the primers A2-F1 (aaacccgggtctagaggatggccgtcatggcg cc) and A2-R1 (cccgcggccgctcacactttacaagctgtgagagac) at 0.5 µM each, 0.2 mM dNTP, and 2 units of cloned Pfu DNA polymerase (Stratagene # 600153). The programme used an initial 10 minutes denaturation step at 95°C followed by 30 cycles of denaturation for 1 minute at 95°C, annealing for 1 min at 50°C, and elongation for 4 minutes at 73°C and a final elongation step at 73°C. The product was purified after electrophoresis on a 1% agarose gel by electro-transfer to GF/C filter, elution from the filter by centrifugation, extraction by Phenol:Chloroform:Isoamylic alcohol (25:24:1) and chromatograhy purified on a Superdex G50 Spincolumn. The purified PCR product and 200 ng pIRES (Clontech #6028-1) was digested with 10 units XbaI and 5 units Not I in a final volume of 20 µl Tris-Acetate buffer (33 mM Tris Acetate pH 7.9, 66 KOAc, 10mM MgOAc, 0.1 mg/ml autoclaved gelatin, 0.5 mM DTT). Digests were purified from an 0.9% agarose/TBE gel using the same technique as described above. The fragment was ligated into pIRES (see Figure 10) using a Rapid DNA Ligation Kit (Roche # 1 635 379) according to the manufacturers instructions. The entire HLA-A^{*}0201 coding sequence (see Figure 11) was cloned between the XmaI or the XbaI site and the NotI site in MCS B. The design of the oligo-nucleotides allows amplification of this sequence from human cDNA. The insert sequence of the resulting plasmid, pEX060, was verified by automated sequencing.

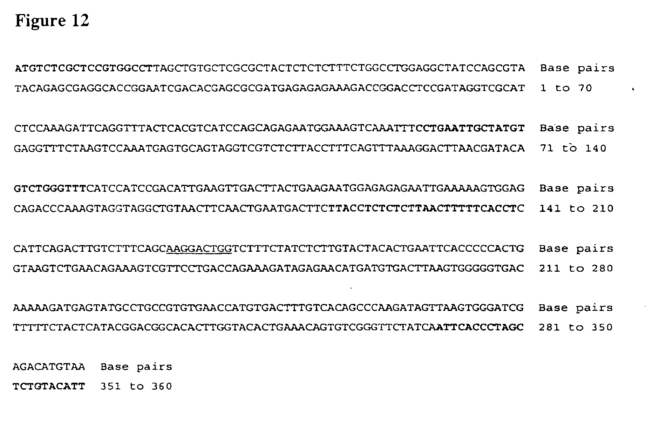

The gene coding for full length wild type β₂m (see Figure 12) was amplified from total T cell RNA by RT-PCR using primers β2m-F2 (cccagctagctcgagatgtctcgctccgtggct) and β2m-R3 (aaacacgcgttacatgtctcgatcccactta) and cloned Xhol-Mlul into pIRES by standard techniques as described above. The insert sequence of the resulting plasmid, pEX066, was verified by automated sequencing.

Different genes coding for full length mutant β2m proteins were prepared by PCR-stitching using primers β2m-F2 + β2m-R2 (tccactttttcaattctctctccatt) on T cell cDNA template for the 5'-end and primers β2m-F3 (cctgaattgctatgtgtctgggtt) and β2m-R3 on plasmid pEX051, pEX052, or pEX56 as template for the 3'-end followed by PCR amplification of the mixed products using β2m-F2 and β2m-R3 and cloned Xho I - Mlu I into pIRES by standard techniques as described above. The insert sequences of the resulting plasmids, pEX067 (K58→E), pEX068 (K58→SES), and pEX069 (K58→R) were verified by automated sequencing.

The HLA-A*02011 fragment from pEX060 was subcloned by standard techniques Xba I - Not I into each of plasmids pEX066, pEX067, pEX068, and pEX069 to give bi-cistronic expression plasmids pEX070 (wt. + HLA-A*02011), pEX071(K58→E + HLA-A*02011), pEX072(K58→SES + HLA-A*02011), and pEX073(K58→R + HLA-A* 02011) respectively.

The vector can be used to induce transient expression of HLA-A*0201 combined with wild type- or mutant β2m in appropriate human target cells. The transfected target cell can be any HLA-A*0201 negative Antigen presenting cell (APC), for example, C1R, T293 or HeLa cells. Transfected and peptide labelled cells can be used as targets for lysis by HLA-A*0201 specific CTLs in a standard chromium release assay, or similar. The vector can also be used to induce transient expression of any wild-type HLA molecule with wild-type or mutant β2m in a similar fashion.

### Example 18 - Assay for Immune-inhibitory effect of HLA-mutant β2m complexes

The ability of target cells transfected with HLA-mutant β2m complexes as described in Example 17 to inhibit *in vitro* CTL activity is determined by the following methodology.

A human T cell line (enriched and selected), recognising the target HLA e.g. HLA-A*0201 is used. A constant number of target cells (5000), expressing the HLA-A*0201-β2m complex, are used and the number of T cells are varied. 'E:T' represents a ratio of numbers of T cells to target cells. Four E:T ratios are tested. The peptide concentrations are varied from 10⁻¹¹ to 10⁻⁷ M. Two sets of experiments probing the mutant HLA-A*0201-mutant β2m complex effect are conducted as follows:
Group 1 - incubation with target cells expressing only the WT HLA-A*0201-β2m complex as a control;
Group 2 - incubation with target cells expressing HLA-A2*0201-mutant -β2m complex.

The assay components for these experiments are: 18 µl 10X peptide; 18 µl PBS; 50 µl containing 5000 target cells; 100 µl containing 5000 -50000 CTL. Results are collected after 2 hours incubation.

The inhibition of CTL activity in cells expressing the HLA A2*0201-mutant -β2m complexes indicates their immune-suppressive activity.

### Example 19 - Inhibition of HLA-A2 restricted CTL clone 5D8 by HLA/ mutant β2m complexes

Target cells (T2 hybrids) were grown in RPMI containing 10% human serum for 5 days. These cells were then incubated with wild-type or mutant β2m protein (K58→R, K58→Y, K58→E, K58→SES) at 300µg/ml in RPMI medium containing 1µM peptide (SLYNTVATL, GAG P17-HIV1) for 2 hours. These cells were then washed and labelled with ⁵¹Cr for 1 hour in the presence of 150µg/ml wild-type or mutant β2m protein. After further washes, the target cells were plated out with CTL at a range of E:T ratios. Wild-type or mutant β2m protein was present at 300µg/ml for the duration of the assay. Supernatants were harvested after 2 hours.

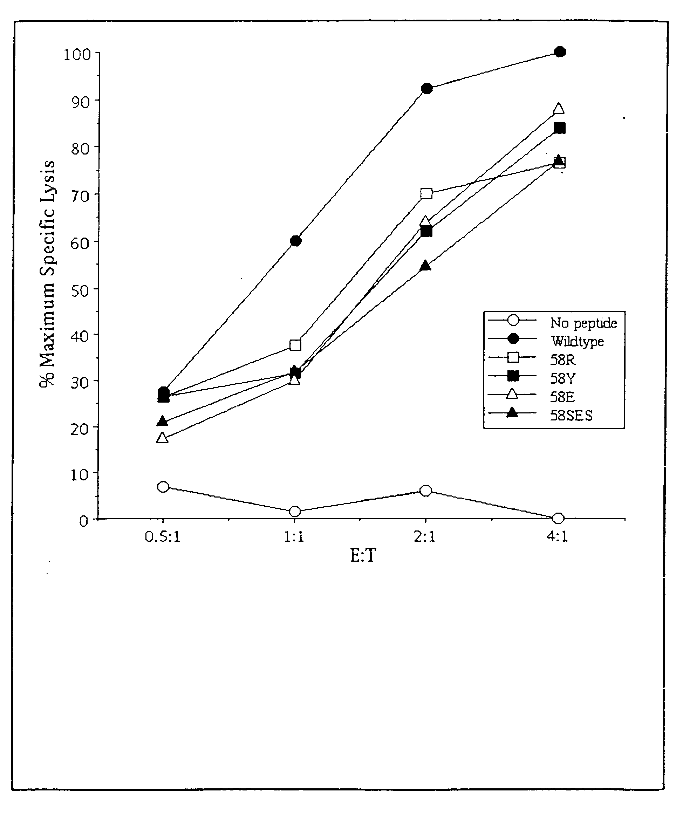

The results are shown in Figure 13, in which it can be seen that all of the mutants tested exhibited an inhibition of CTL activity at E:T ratios of 1:1 or greater.

### Example 20 - Inhibition of HLA-A2 restricted parvovirus B19 specific CTL line by HLA/mutant β2m complexes

Target cells (T2 hybrids) were grown in RPMI containing 10% bovine serum for 5 days. These cells were then washed and labelled with ⁵¹Cr for 1 hour. The target cells were then incubated with either wild-type β2m or mutant β2m (K58→E) at 30µg/ml in RPMI medium containing 100 nM peptide (epitope is EADVQQWLTW) for 15 minutes. The target cells were plated out with CTL (CTL line) at a ratio of 22:1 (E:T ratio). Wild-type (WT) β2m or mutant β2m was present at 30µg/ml for the duration of the assay. Supernatants were harvested after 4 hours.

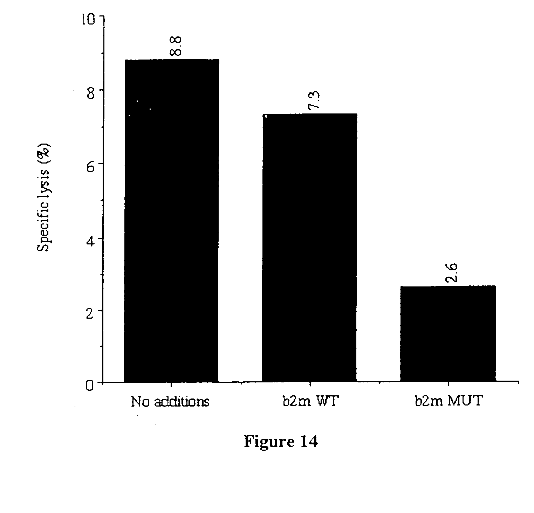

The results are shown in Figure 14. The addition of mutant β2m protein resulted in a specific lysis of 2.6% compared to 7.3% in the presence of WT β2m protein. This demonstrates the ability of mutant β2m protein to inhibit CTL activity.

### Example 21- Test for Immunogenicity of mutant β-2-microglobulin

Mutant β-2-microglobulin molecules could potentially induce an immune response either by antibodies and/or T cells. The introduction of mutation(s) in the β-2-microglobulin protein could potentially introduce a conformational change in the protein structure, which would be recognised by antibodies as a structural foreign antigen or, alternatively, enzymatic degradation of mutant β-2-microglobulin could produce peptides not normally presented by self MHC molecules. These mutant peptides would then be recognised as foreign and induce a cellular immune response. Therefore, mutants can be tested in a transgenic rat model expressing human MHC class I molecule (HLA-B27 heavy chain + β-2-microglobulin) as follows.
1. Inject transgenic rats with 3-4mg mutant beta-2-microglobulin.
2. Collect serum from rats after 21 days.
3. Analyse serum for the production of anti-mutant β-2-microglobulin antibodies by an ELISA. The procedure for this ELISA is: a. Bind mutant beta-2-microglobulin to bottom of well; b. Add serum from transgenic rat, which has been treated with mutant beta-2-microglobulin; c. wash three times with 200µL of wash buffer; d.add the appropriate concentration of conjugated anti-rat antibody; e. wash three times with 200µL of wash buffer; f. add 100µL detection reagent (Alkaline Phosphatase, substrate pNPP) and read absorbance at 405nm. Absorbance readings above negative control readings will indicate, that anti-mutant β-2-microglobulin antibodies are present in the serum.

### Example 22 - Inhibition of CTL priming and activity by mutant HLA/β2m complexes in vivo.

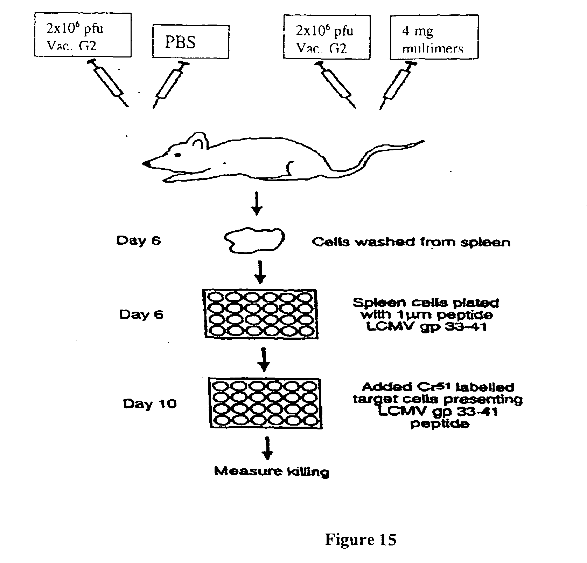

The ability of multimers of class I MHC containing mutant β2m to inhibit a cellular immune response *in vivo* is tested by three experiments in mouse, assaying the influence of mutant β2m on the response to an epitope encoded in a vaccinia virus (See Figure 15).

### Materials and reagents:

Mice: In all three experiments, mice of strain Black 6 (C57BL/6 wildtype laboratory mouse strain) are used.
Virus: Vaccinia virus strain G2 (vvG2) is a recombinant virus strain. Infection of cells with Vaccinia virus generally results in the MHC restricted presentation of a number of vaccinia peptide epitopes which are poorly characterised. Infection with strain vvG2, however, also results in the presentation of a peptide epitope, corresponding to amino acids 33-41 (sequence in one-letter code: KAVYNFATC) from the LCM virus G2 glycoprotein. In mice, the G2 epitope is presented by the MHC molecule D^{b}.
Target cells : MC57 antigen presenting cells (Leist, et al (1987) J Immunol 138: 2278-81) express mouse MHC molecules K^{b} and D^{b}.

### Experiment 1:

A control group of mice are injected, intravenously, at Day 0 with 2x10⁶ pfu (plaque forming units) vaccinia virus strain G2 plus 200 µl phosphate buffered saline (PBS, standard physiological saline buffer). A test group of mice are also injected, intravenously, at Day 0 with 2x 10⁶ pfu Vaccinia virus strain G2 plus 200 µl mutant MHC multimers (20mg/ml) in PBS buffer.

To assay CTL activity, a modification of a previously described procedure can be used (Leist, *et al* (1987) *J Immunol* **138**: 2278-81). On Day 6, the spleens are removed from the mice and lymphocytes washed out. Spieen cells are cultured in 24-well plates. Synthetic peptide corresponding to the G2 33-41 epitope is added to the wells and lymphocytes allowed to proliferate for four days. On Day 10, T cells are counted and CTL killing assays are performed as follows. Chromium⁵¹ -labelled MC57 cells are prepulsed with G2 33-41 peptide for one hour, then mixed with the cultured mouse lymphocytes at a range of effector to target ratios. Chromium⁵¹ counts from wells of MC57 cells lysed by addition of detergent are set at 100% lysis.

### Experiment 2:

The control and test group of mice are treated identically to those in Experiment 1 except that injections are performed intraperitoneally. CTL activity is assessed as described in Experiment 1.

### Experiment 3:

A control group of mice are injected intraperitoneally with 200 µl phosphate buffered saline. This dose is administered five hours prior to infection vaccinia virus strain G2, and a further injection 24 hours after the first one. A test group of mice is injected intraperitoneally with MHC multimers containing mutant β2m protein (4 mg) in PBS buffer. This dose is administered five hours prior to infection vaccinia virus strain G2, and a further injection 24 hours after the first one.

CTL activity is assessed as described in Experiment 1.

### Example 23 - Production of multimeric peptide-MHC tetramers and beads containing modified β₂m

Biotinylated peptide-MHC proteins containing modified β₂m are produced as in Example 13 (O'Callaghan, *et al. Anal Biochem* **266**(1): 9-15 (1999)). Tetrameric complexes are obtained by adding avidin, streptavidin or a modified avidin (eg. extravidin) to the biotinylated protein at a 1:4 molar ratio. The amount of avidin required may be calculated from a protein assay of the biotinylated peptide-MHC complex. Polystyrene beads coated with streptavidin or modified avidin may be obtained from Molecular Probes. Such beads are incubated with a saturating concentration of biotinylated peptide-MHC complex for 5 minutes, and are then washed three times in PBS to remove excess unbound protein.

### Example 24 - Use of peptide-MHC multimers to induce activation-induced cell death (AICD) in specific T cell populations.

CTL are incubated with target cells at effector:target (E:T) ratios varying from 0.1:1 to 10:1, or with multimeric beads (5 beads / cell) or with peptide-MHC tetramers (1-50 µg/10⁶ cells) at 37°C for 5 - 30 minutes. Cell-induced apoptosis is measured by Annexin-V staining or TUNEL assay: CTL are labelled with anti-CD8, B-cell targets are labelled with anti-CD19 and then CTL are stained with Annexin-V-FITC or TUNEL-FITC according to manufacturer's protocol. Tetramer or particle-induced apoptosis is measured by JAM assay. Briefly, the CTL are cultured with ³H-TdR (1µCi/ml) for 12-24 hours, washed 3 times, and then cultured with the multimeric beads for another 12 hours. DNA fragmentation was determined by ³H-Thymidine release.

### Example 25 - A DNA plasmid vector for expression of peptide-β2microglobulin fusions in E.coli.

This example describes the design and construction of a DNA plasmid vector for expression of peptide-β2microglobulin fusions in *E.coli.* The vector was designed so that the peptide to be expressed can be easily changed by inserting a pair of synthetic DNA oligonucleotides encoding the new peptide sequence.

A PCR reaction was performed with cDNA generated from human cytotoxic T lymphocytes (CTL) as a template. The primers used had the following sequences:

The PCR reaction generated a DNA fragment comprising the sequence encoding human β₂microglobulin as expressed on the cell surface, i.e. without the signal peptide. The fragment also contained sequence 5' of the β2microglobulin gene which encodes a linker fusion sequence. The fragment was cloned into the DNA expression vector pGMT7 (Studier, *et al, Methods Enzymol* (1990) **185:** 60-89 Issn: 0076-6879) using the unique BamHI (GGA TCC ) and HindIII (GAA TTC) restriction sites which are underlined in the primer sequences above.

The resulting DNA plasmid was then modified by cloning a pair of synthetic DNA oligonucleotides into the unique NdeI and BamHI sites. The sequences of the DNA oligonucleotides were as follows:
5' T ATG GGT ATT TTA GGA TTT GTT TTT ACA TTA G 3' (coding strand)

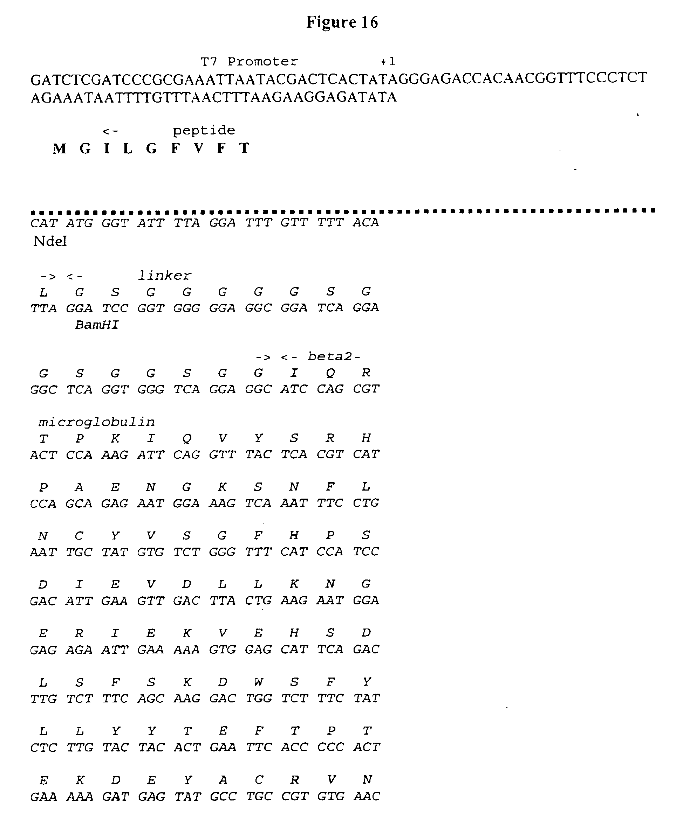

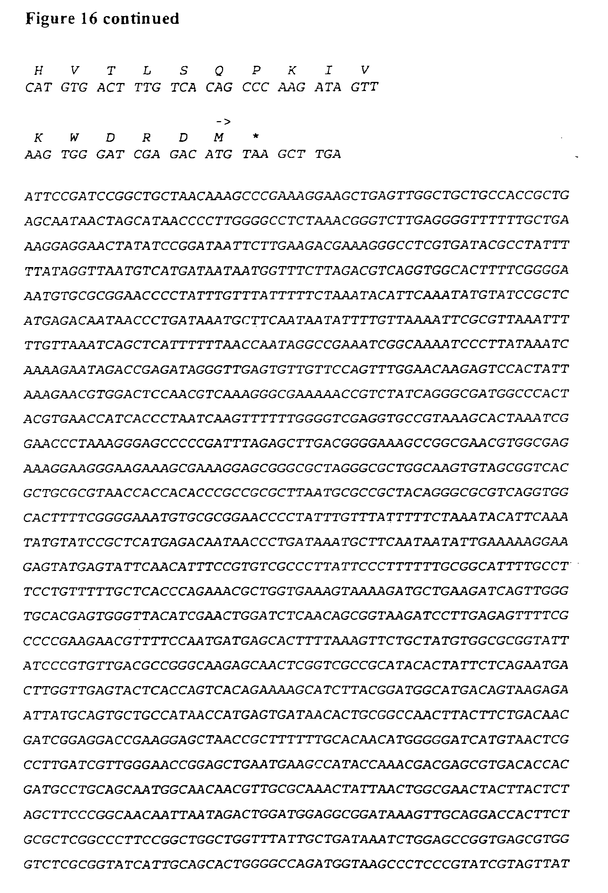

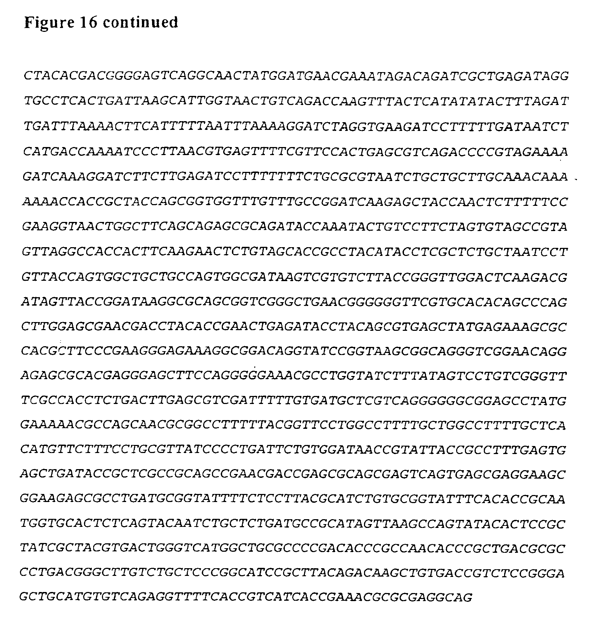

The resulting DNA plasmid, pBJ 196, can be used for expression of a polypeptide which is a fusion of three elements: a peptide corresponding to an antigenic peptide from influenza virus ('flu matrix peptide) which is known to be presented by HLA-A0201, a flexible linker sequence mainly comprised of Glycine and Serine residues, and β2microglobulin. The DNA sequence of plasmid pBJ196 is shown in Figure 16. The DNA sequence of pBJ 196 was verified by dideoxy sequencing. The amino acid sequence of the fusion protein is indicated above the DNA sequence of the relevant region. Also indicated are the start site of transcription ('+1') and the restriction sites that can be used to substitute the peptide coding sequence for DNA cassettes encoding other antigen peptides. The vector pBJ 196 contains a T7 promoter for high-level expression in a suitable *E.coli* strain such as BL21DE3pLysS or BL21DE3pLysE (Novagen).

### Example 26 - Mutation of peptide-β2microglobulin fusion plasmid vector to produce peptide-mutant β2microglobulin fusions

The DNA plasmid, pBJ 196, produced as described in Example 25 can be mutated to provide plasmids coding for peptide-mutant β₂microglobulin fusions. The mutation is effected by applying the primers and methods described in Examples 1-12 to plasmid pBJ196.

### Example 27 - A DNA plasmid vector for expression of an HLA-A2 null peptide-mutant β2microglobulin fusion in E.coli.

This example describes the design and construction of a DNA plasmid vector for expression of a HLA-A2 null peptide-mutant β₂microglobulin fusion.

The plasmid vector produced in Example 26 can be modified by restriction enzyme digestion with enzymes NdeI and BamHI and insertion of the synthetic oligonucleotide pair:
5' T ATG GCA CTG GCT GCG GCA GCC GCA GCG GTT G 3' (coding strand)

This will generate a plasmid coding for a peptide, fused to mutant β2microglobulin, with the sequence ALAAAAAAV. This peptide contains Alanine residues in all positions except positions 2 and 9 which are optimised for anchoring the peptide to the HLA-A*0201 heavy chain.

## Claims

1. A multimer comprising a plurality of class I Major Histocompatibility Complexes (MHCs) at least one of which has a modified β₂-microglobulin whose binding to CD8 as measured by surface plasmon resonance is inhibited, wherein the mutated residue is one or more of Serine₅₇, Lysine₅₈, Aspartate₅₉, Tryptophan₆₀ and Serine₆₁.

2. A multimer as claimed in claim 1, wherein the modified β₂-microglobulin differs from wild type β₂-microglobulin by virtue of one or more deletion or substitution mutations at said any one or more of Serine₅₇, Lysine₅₈, Aspartate₅₉, Tryptophan₆₀ and Serine₆₁ or insertion mutations immediately before or after any of said Serine₅₇, Lysine₅₈, Aspartate₅₉, Tryptophan₆₀ and Serine₆₁.

3. A multimer as claimed in claim 2, wherein the modified β₂-microglobulin differs from wild type β₂-microglobulin by virtue of one or more mutated residues at said one or more of Serine₅₇, Lysine₅₈, Aspartate₅₉, Tryptophan₆₀ and Serine₆₁which sterically and/or electrostatically inhibit or impair the binding of CD8 to the MHC.

4. A multimer as claimed in claim 1, 2 or 3, wherein the modified β₂-microglobulin is derived from human β₂-microglobulin.

5. A multimer as claimed in claim 1, wherein the mutated residue is mutated to Cysteine which is derivatised.

6. A multimer as claimed in claim 1 or claim 5, wherein Lysine₅₈ is replaced with Arginine, Glutamate, Aspartate, Serine, Tyrosine, Tryptophan, Cysteine, Serine-Glutamate-Serine, or Glycine-Arginine-Glycine.

7. A multimer as claimed in claim 6, wherein Lysine₅₈ is replaced with Arginine, Glutamate or Aspartate.

8. A multimer as claimed in claim 1, wherein Aspartate₅₉ is replaced with Glycine-Glutamate-Glycine.

9. A multimer as claimed in claim 5, wherein Tryptophan₆₀ is replaced with Glycine.

10. A multimer as claimed in claim 1, wherein Lysine₅₈ is replaced with Arginine, and Tryptophan₆₀ is replaced with Glycine.

11. A multimer as claimed in any preceding claim, comprising an MHC associated with a specific disorder.

12. A multimer as claimed in any preceding claim, comprising a peptide associated with a specific disorder.

13. A multimer as claimed in any one of claims 1 to 10, comprising a peptide not associated with a specific disorder.

14. A multimer as claimed in claim 13, comprising a peptide selected from a random pool of peptides or which presents substantially no T cell recognition features or in which one or more T cell recognition features are randomly present.

15. A multimer as claimed in claim 12, 13 or 14, wherein the peptide is attached to the β₂m with a linker.

16. A multimer as claimed in any preceding claim, wherein each MHC molecule is associated via a linker molecule.

17. A multimer as claimed in claim 16, wherein said linker molecule is neutravidin, avidin, streptavidin, extravidin, Igm or human complement factor C1Q.

18. A multimer as claimed in any preceding claim, wherein each MHC molecule is attached to a liposome or particle.

19. An in-vtro method of inhibiting a CD8⁺ T cell response, comprising exposing the CD8⁺ T cell to a multimer as claimed in any one of claims 1 to 18

20. A multimer as claimed in any one of claims 1 to 18 for use in medicine.

21. The use of a multimer as claimed in any one of claims 1 to 18 in the manufacture of a medicament for inhibiting CD8⁺ T cell response.

22. A method for selecting/isolating T cells comprising bringing a sample containing T cells into contact with a multimer as claimed in any one of claims 1 to 18, and selecting/isolating T cells which bind to the multimer.

## Patentansprüche

1. Multimer, umfassend eine Vielzahl von Klasse I Haupt-Histokompatibilitäts-Komplexen (MHCs), wobei mindestens einer davon ein modifiziertes β₂-Mikroglobulin aufweist, dessen Bindung an CD8, gemessen durch Oberflächenplasmonresonanz, inhibiert ist, wobei der mutierte Rest ein oder mehr ist gewählt aus Serin₅₇, Lysin₅₈, Aspartat₅₉, Tryptophan₆₀ und Serin₆₁.

2. Multimer gemäß Anspruch 1, wobei sich das modifizierte β₂-Mikroglobulin vom Wildtyp-β₂-Mikroglobulin durch ein oder mehr Deletions- oder Substitutionsmutationen an dem ein oder mehr von Serin₅₇, Lysin₅₈, Aspartat₅₉, Tryptophan₆₀ und Serin₆₁ oder durch Insertionsmutationen direkt vor oder nach einem von Serin₅₇, Lysin₅₈, Aspartat₅₉, Tryptophan₆₀ und Serin₆₁ unterscheidet.

3. Multimer gemäß Anspruch 2, wobei sich modifizierte β₂-Mikroglobulin vom Wildtyp-β₂-Mikroglobulin durch ein oder mehr mutierte Reste an den ein oder mehr aus Serin₅₇, Lysin₅₈, Aspartat₅₉, Tryptophan₆₀ und Serin₆₁ unterscheidet, die sterisch und/oder elektrostatisch die Bindung von CD8 an den MHC inhibieren oder beeinträchtigen.

4. Multimer gemäß einem der Ansprüche 1, 2 oder 3, wobei das modifizierte β₂-Mikroglobulin von menschlichem β₂-Mikroglobulin abstammt.

5. Multimer gemäß Anspruch 1, wobei der mutierte Rest zu Cystein mutiert ist, das derivatisiert ist.

6. Multimer gemäß einem der Ansprüche 1 oder 5, wobei Lysin₅₈ durch Arginin, Glutamat, Aspartat, Serin, Tyrosin, Tryptophan, Cystein, Serin-Glutamat-Serin oder Glycin-Arginin-Glycin ersetzt ist.

7. Multimer gemäß Anspruch 6, wobei Lysin₅₈ durch Arginin, Glutamat oder Aspartat ersetzt ist.

8. Multimer gemäß Anspruch 1, wobei Aspartat₅₉ durch Glycin-Glutamat-Glycin ersetzt ist.

9. Multimer gemäß Anspruch 5, wobei Tryptophan₆₀ durch Glycin ersetzt ist.

10. Multimer gemäß Anspruch 1, wobei Lysin₅₈ durch Arginin und Tryptopohan₆₀ durch Glycin ersetzt ist.

11. Multimer gemäß einem der vorstehenden Ansprüche, umfassend einen MHC, assoziiert mit einer spezifischen Erkrankung.

12. Multimer gemäß einem der vorstehenden Ansprüche, umfassend ein Peptid, assoziiert mit einer spezifischen Erkrankung.

13. Multimer gemäß einem der Ansprüche 1 bis 10, umfassend ein Peptid, das nicht mit einer spezifischen Erkrankung assoziiert ist.

14. Multimer gemäß Anspruch 13, umfassend ein Peptid, gewählt aus einem zufälligen Pool von Peptiden, oder das im wesentlichen keine T-Zell-Erkennungsmerkmale präsentiert oder worin ein oder mehr T-Zell-Erkennungsmerkmale zufällig vorliegen.

15. Multimer gemäß einem der Ansprüche 12, 13 oder 14, wobei das Peptid an β₂m mit einem Linker gebunden ist.

16. Multimer gemäß einem der vorstehenden Ansprüche, worin jedes MHC-Molekül über ein Linkermolekül assoziiert ist.

17. Multimer gemäß Anspruch 16, wobei das Linkermolekül Neutravidin, Avidin, Streptavidin, Extravidin, IgM oder menschlicher Komplementfaktor C1Q ist.

18. Multimer gemäß einem der vorstehenden Ansprüche, worin jedes MHC-Molekül an ein Liposom oder Teilchen angebunden ist.

19. In-vitro-Verfahren zur Inhibition einer CD8⁺ T-Zellreaktion, umfassend das Aussetzen der CD8⁺ T-Zelle gegenüber einem Multimer gemäß einem der Ansprüche 1 bis 18.

20. Multimer gemäß einem der Ansprüche 1 bis 18 zur Verwendung in der Medizin.

21. Verwendung eines Multimers gemäß einem der Ansprüche 1 bis 18 bei der Herstellung eines Medikaments zur Inhibition der CD8⁺ T-Zellreaktion.

22. Verfahren zur Selektion/Isolation von T-Zellen, umfassend das Kontaktieren einer Probe, enthaltend T-Zellen mit einem Multimer gemäß einem der Ansprüche 1 bis 18, und Selektion/Isolation von T-Zellen, die an das Multimer binden.

## Revendications

1. Multimère comprenant une pluralité de complexes majeurs d'histocompatibilité classe I (CMH) dont au moins un présente une β₂-microglobuline modifiée dont la liaison à CD8, telle qu'elle est mesurée par la résonance plasmonique de surface, est inhibée, le résidu muté étant un ou plusieurs éléments choisis parmi la sérine₅₇, la lysine₅₈, l'aspartate₅₉, le tryptophane₆₀ et la sérine₆₁.

2. Multimère selon la revendication 1, dans lequel la β₂₋ microglobuline modifiée diffère de la β₂-microglobuline de type sauvage du fait d'une ou plusieurs mutations de substitution ou délétion survenues au niveau de l'un quelconque ou de plusieurs desdits éléments choisis parmi la sérine₅₇, la lysine₅₈, l'aspartate₅₉, le tryptophane₆₀ et la sérine₆₁.ou du fait de mutations d'insertion précédant immédiatement ou intervenant juste après l'un quelconque desdits éléments choisis parmi la sérine₅₇, la lysine₅₈, l'aspartate₅₉, le tryptophane₆₀ et la sérine₆₁ .

3. Multimère selon la revendication 2, dans lequel la β₂-microglobuline modifiée diffère de la β₂-microglobuline de type sauvage du fait d'un ou plusieurs résidus mutés au niveau d'un ou de plusieurs éléments choisis parmi la sérine₅₇, la lysine₅₈, l'aspartate₅₉, le tryptophane₆₀ et la sérine₆₁ qui inhibent ou altèrent stériquement et/ou électrostatiquement la liaison de CD8 au CMH.

4. Multimère selon la revendication 1, 2 ou 3, dans lequel la β₂-microglobuline modifiée est dérivée de la β₂-microglobuline humaine.

5. Multimère selon la revendication 1, dans lequel le résidu muté est muté en une cystéine dérivée.

6. Multimère selon la revendication 1 ou 5, dans lequel la lysine₅₈ est remplacée par l'arginine, le glutamate, l'aspartate, la sérine, la tyrosine, le tryptophane, la cystéine, la sérine-glutamate-sérine, ou la glycine-arginine-glycine.

7. Multimère selon la revendication 6, dans lequel la lysine₅₈ est remplacée par l'arginine, le glutamate ou l'aspartate.

8. Multimère selon la revendication 1, dans lequel l'aspartate₅₉ est remplacé par la glycine-glutamate-glycine.

9. Multimère selon la revendication 5, dans lequel le tryptophane₆₀ est remplacé par la glycine.

10. Multimère selon la revendication 1, dans lequel la lysine₅₈ est remplacée par l'arginine, et le tryptophane₆₀ est remplacé par la glycine.

11. Multimère selon l'une quelconque des revendications précédentes, comprenant un CMH associé à un trouble spécifique.

12. Multimère selon l'une quelconque des revendications précédentes, comprenant un peptide associé à un trouble spécifique.

13. Multimère selon l'une quelconque des revendications 1 à 10, comprenant un peptide non associé à un trouble spécifique.

14. Multimère selon la revendication 13, comprenant un peptide choisi dans un regroupement aléatoire de peptides ou qui ne présente substantiellement aucune caractéristique de reconnaissance des lymphocytes T ou dans lequel une ou plusieurs caractéristiques de reconnaissance des lymphocytes T sont présentes de façon aléatoire.

15. Multimère selon l'une quelconque des revendications 12, 13 ou 14, dans lequel le peptide est attaché à la β₂-microglobuline par l'intermédiaire d'un lieur.

16. Multimère selon l'une quelconque des revendications précédentes, dans lequel chaque molécule de CMH est associée au moyen d'une molécule de liaison.

17. Multimère selon la revendication 16, dans lequel ladite molécule de liaison est la neutravidine, l'avidine, la streptavidine, l'extravidine, l'Igm ou le facteur du complément humain C1Q.

18. Multimère selon l'une quelconque des revendications précédentes, dans lequel chaque molécule CMH est fixée à un liposome ou à une particule.

19. Procédé *in vitro* d'inhibition de la réponse des lymphocytes T CD8⁺ comprenant l'exposition du lymphocyte T CD8⁺ à un multimère selon l'une quelconque des revendications 1 à 18.

20. Multimère selon l'une quelconque des revendications 1 à 18 destiné à une utilisation dans le domaine médical.

21. Utilisation d'un multimère selon l'une quelconque des revendications 1 à 18 dans la fabrication d'un médicament permettant d'inhiber la réponse des lymphocytes T CD8⁺.

22. Procédé permettant de sélectionner et d'isoler des lymphocytes T comprenant la mise en contact d'un échantillon contenant des lymphocytes T et d'un multimère selon l'une quelconque des revendication 1 à 18, et de sélectionner/isoler des lymphocytes T qui se lient à ce multimère.


### SEQUENCE LISTING

<110> Avidex Limited
   Boulter, Jonathan M
   Jakobsen, Bent K
<120> Substances
<130> P32645WO
<140> PCT/GB02/02866
   <141> 2002-06-19
<150> GB 0115071.3
   <151> 2001-06-20
<160> 65
<170> PatentIn version 3.2
<210> 1
   <211> 53
   <212> DNA
   <213> Artificial
<220>
   <223> Primer
<400> 1
   ggggggcata tgattcaaag aactccaaaa attcaggttt actcacgtca tcc 53
<210> 2
   <211> 36
   <212> DNA
   <213> Artificial
<220>
   <223> Primer
<400> 2
   gggggaagct tacatgtctc gatcccactt aactat 36
<210> 3
   <211> 34
   <212> DNA
   <213> Artificial
<220>
   <223> Primer
<400> 3
   aaacccgggt ctagaggatg gccgtcatgg cgcc 34
<210> 4
   <211> 36
   <212> DNA
   <213> Artificial
<220>
   <223> Primer
<400> 4
   cccgcggccg ctcacacttt acaagctgtg agagac 36
<210> 5
   <211> 33
   <212> DNA
   <213> Artificial
<220>
   <223> Primer
<400> 5
   cccagctagc tcgagatgtc tcgctccgtg gct 33
<210> 6
   <211> 31
   <212> DNA
   <213> Artificial
<220>
   <223> Primer
<400> 6
   aaacacgcgt tacatgtctc gatcccactt a 31
<210> 7
   <211> 26
   <212> DNA
   <213> Artificial
<220>
   <223> Primer
<400> 7
   tccacttttt caattctctc tccatt 26
<210> 8
   <211> 24
   <212> DNA
   <213> Artificial
<220>
   <223> Primer
<400> 8
   cctgaattgc tatgtgtctg ggtt 24
<210> 9
   <211> 9
   <212> PRT
   <213> Artificial
<220>
<223> Peptide
<400> 9
   Ser Leu Tyr Asn Thr Val Ala Thr Leu
   1 5
<210> 10
   <211> 10
   <212> PRT
   <213> Artificial
<220>
   <223> Peptide
<400> 10
   Glu Ala Asp Val Gln Gln Trp Leu Thr Trp
   1 5 10
<210> 11
   <211> 9
   <212> PRT
   <213> Unknown
<220>
   <223> LCM virus epitope
<400> 11
   Lys Ala Val Tyr Asn Phe Ala Thr Cys
   1 5
<210> 12
   <211> 78
   <212> DNA
   <213> Artificial
<220>
   <223> Primer
<400> 12
   ggggggggat ccggtggggg aggcggatca ggaggctcag gtgggtcagg aggcatccag 60
   cgtactccaa agattcag 78
<210> 13
   <211> 47
   <212> DNA
   <213> Artificial
<220>
   <223> Primer
<400> 13
   gggggggaat tcaagcttac atgtctcgat cccacttaac tatcttg 47
<210> 14
   <211> 32
   <212> DNA
   <213> Artificial
<220>
   <223> oligonucleotide
<400> 14
   tatgggtatt ttaggatttg tttttacatt ag 32
<210> 15
   <211> 34
   <212> DNA
   <213> Artificial
<220>
   <223> Primer
<400> 15
   gatcctaatg taaaaacaaa tcctaaaata ccca 34
<210> 16
   <211> 32
   <212> DNA
   <213> Artificial
<220>
   <223> Primer
<400> 16
   tatggcactg gctgcggcag ccgcagcggt tg 32
<210> 17
   <211> 34
   <212> DNA
   <213> Artificial
<220>
   <223> Primer
<400> 17
   gatccaaccg ctgcggctgc cgcagccagt gcca 34
<210> 18
   <211> 9
   <212> PRT
   <213> Artificial
<220>
   <223> mutant peptide
<400> 18
   Ala Leu Ala Ala Ala Ala Ala Ala Val
   1               5
<210> 19
   <211> 303
   <212> DNA
   <213> Homo sapiens
<220>
   <221> CDS
   <222> (1)..(303)
<400> 19
   atg atc cag cgt act cca aag att cag gtt tac tca cgt cat cca gca        48
   gag aat gga aag tca aat ttc ctg aat tgc tat gtg tct ggg ttt cat        96
   cca tcc gac att gaa gtt gac tta ctg aag aat gga gag aga att gaa       144
   aaa gtg gag cat tca gac ttg tct ttc agc aag gac tgg tct ttc tat       192
   ctc ttg tac tac act gaa ttc acc ccc act gaa aaa gat gag tat gcc       240
   tgc cgt gtg aac cat gtg act ttg tca cag ccc aag ata gtt aag tgg       288
   gat cga gac atg taa                                                   303
<210> 20
   <211> 100
   <212> PRT
   <213> Homo sapiens
<400> 20
<210> 21
   <211> 303
   <212> DNA
   <213> Homo sapiens
<400> 21
   ttacatgtct cgatcccact taactatctt gggctgtgac aaagtcacat ggttcacacg      60
   gcaggcatac tcatcttttt cagtgggggt gaattcagtg tagtacaaga gatagaaaga     120
   ccagtccttg ctgaaagaca agtctgaatg ctccactttt tcaattctct ctccattctt     180
   cagtaagtca acttcaatgt cggatggatg aaacccagac acatagcaat tcaggaaatt     240
   tgactttcca ttctctgctg gatgacgtga gtaaacctga atctttggag tacgctggat     300
   cat                                                                   303
<210> 22
   <211> 69
   <212> DNA
   <213> Artificial
<220>
   <223> mutant sequence
<220>
   <221> CDS
   <222> (1)..(69)
<400> 22
   aaa gtg gag cat tca gac ttg tct ttc tct gag gac tgg tct ttc tat       48
   Lys Val Glu His Ser Asp Leu Ser Phe Ser Glu Asp Trp Ser Phe Tyr
   1               5                   10                  15
   ctc ttg tac tac act gaa ttc                                           69
   Leu Leu Tyr Tyr Thr Glu Phe
               20
<210> 23
   <211> 23
   <212> PRT
<213> Artificial
<220>
   <223> mutant sequence
<400> 23
   Lys Val Glu His Ser Asp Leu Ser Phe Ser Glu Asp Trp Ser Phe Tyr
   1               5                   10                  15
   Leu Leu Tyr Tyr Thr Glu Phe
               20
<210> 24
   <211> 69
   <212> DNA
   <213> Artificial
<220>
   <223> mutant sequence
<400> 24
   gaattcagtg tagtacaaga gatagaaaga ccagtcctca gagaaagaca agtctgaatg      60
   ctccacttt                                                              69
<210> 25
   <211> 69
   <212> DNA
   <213> Artificial
<220>
   <223> mutant sequence
<220>
   <221> CDS
   <222> (1)..(69)
<400> 25
   aaa gtg gag cat tca gac ttg tct ttc tct tct gac tgg tct ttc tat      48
   Lys Val Glu His Ser Asp Leu Ser Phe Ser Ser Asp Trp Ser Phe Tyr
   1               5                   10                  15
   ctc ttg tac tac act gaa ttc                                          69
   Leu Leu Tyr Tyr Thr Glu Phe
               20
<210> 26
   <211> 23
   <212> PRT
   <213> Artificial
<220>
   <223> mutant sequence
<400> 26
   Lys Val Glu His Ser Asp Leu Ser Phe Ser Ser Asp Trp Ser Phe Tyr
   1               5                   10                  15
   Leu Leu Tyr Tyr Thr Glu Phe
               20
<210> 27
   <211> 69
   <212> DNA
   <213> Artificial
<220>
   <223> mutant sequence
<400> 27
   gaattcagtg tagtacaaga gatagaaaga ccagtcagaa gagaaagaca agtctgaatg    60
   ctccacttt                                                            69
<210> 28
   <211> 69
   <212> DNA
   <213> Artificial
<220>
   <223> mutant sequence
<220>
   <221> CDS
   <222> (1)..(69)
<400> 28
   aaa gtg gag cat tca gac ttg tct ttc tct cgc gac tgg tct ttc tat      48
   Lys Val Glu His Ser Asp Leu Ser Phe Ser Arg Asp Trp Ser Phe Tyr
   1               5                   10                  15
   ctc ttg tac tac act gaa ttc                                          69
   Leu Leu Tyr Tyr Thr Glu Phe
               20
<210> 29
   <211> 23
   <212> PRT
   <213> Artificial
<220>
   <223> mutant sequence
<400> 29
   Lys Val Glu His Ser Asp Leu Ser Phe Ser Arg Asp Trp Ser Phe Tyr
   1               5                   10                  15
   Leu Leu Tyr Tyr Thr Glu Phe
               20
<210> 30
   <211> 69
   <212> DNA
   <213> Artificial
<220>
   <223> mutant sequence
<400> 30
   gaattcagtg tagtacaaga gatagaaaga ccagtcgcga gagaaagaca agtctgaatg      60
   ctccacttt                                                              69
<210> 31
   <211> 69
   <212> DNA
   <213> Artificial
<220>
   <223> mutant sequence
<220>
   <221> CDS
   <222> (1)..(69)
<400> 31
   aaa gtg gag cat tca gac ttg tct ttc tct tac gac tgg tct ttc tat        48
   Lys Val Glu His Ser Asp Leu Ser Phe Ser Tyr Asp Trp Ser Phe Tyr
   1               5                   10                  15
   ctc ttg tac tac act gaa ttc                                            69
   Leu Leu Tyr Tyr Thr Glu Phe
               20
<210> 32
   <211> 23
   <212> PRT
   <213> Artificial
<220>
   <223> mutant sequence
<400> 32
   Lys Val Glu His Ser Asp Leu Ser Phe Ser Tyr Asp Trp Ser Phe Tyr
   1               5                   10                  15
   Leu Leu Tyr Tyr Thr Glu Phe
               20
<210> 33
   <211> 69
   <212> DNA
   <213> Artificial
<220>
   <223> mutant sequence
<400> 33
   gaattcagtg tagtacaaga gatagaaaga ccagtcgtaa gagaaagaca agtctgaatg      60
   ctccacttt                                                              69
<210> 34
   <211> 69
   <212> DNA
   <213> Artificial
<220>
   <223> mutant sequence
<220>
   <221> CDS
   <222> (1)..(69)
<400> 34
   aaa gtg gag cat tca gac ttg tct ttc agc tgt gac tgg tct ttc tat      48
   Lys Val Glu His Ser Asp Leu Ser Phe Ser Cys Asp Trp Ser Phe Tyr
   1               5                   10                  15
   ctc ttg tac tac act gaa ttc                                          69
   Leu Leu Tyr Tyr Thr Glu Phe
               20
<210> 35
   <211> 23
   <212> PRT
   <213> Artificial
<220>
   <223> mutant sequence
<400> 35
   Lys Val Glu His Ser Asp Leu Ser Phe Ser Cys Asp Trp Ser Phe Tyr
   1               5                   10                  15
   Leu Leu Tyr Tyr Thr Glu Phe
               20
<210> 36
   <211> 69
   <212> DNA
   <213> Artificial
<220>
   <223> mutant sequence
<400> 36
   gaattcagtg tagtacaaga gatagaaaga ccagtcacag ctgaaagaca agtctgaatg     60
   ctccacttt                                                             69
<210> 37
   <211> 75
   <212> DNA
   <213> Artificial
<220>
   <223> mutant sequence
<220>
   <221> CDS
   <222> (1)..(75)
<400> 37
   aaa gtg gag cat tca gac ttg tct ttc agc tct gag tct gac tgg tct       48
   Lys Val Glu His Ser Asp Leu Ser Phe Ser Ser Glu Ser Asp Trp Ser
   1               5                   10                  15
   ttc tat ctc ttg tac tac act gaa ttc                                   75
   Phe Tyr Leu Leu Tyr Tyr Thr Glu Phe
               20                  25
<210> 38
   <211> 25
   <212> PRT
   <213> Artificial
<220>
   <223> mutant sequence
<400> 38
   Lys Val Glu His Ser Asp Leu Ser Phe Ser Ser Glu Ser Asp Trp Ser
   1               5                   10                  15
   Phe Tyr Leu Leu Tyr Tyr Thr Glu Phe
               20                  25
<210> 39
   <211> 75
   <212> DNA
   <213> Artificial
<220>
   <223> mutant sequence
<400> 39
   gaattcagtg tagtacaaga gatagaaaga ccagtcagac tcagagctga aagacaagtc      60
   tgaatgctcc acttt                                                       75
<210> 40
   <211> 69
   <212> DNA
   <213> Artificial
<220>
   <223> mutant sequence
<220>
   <221> CDS
   <222> (1)..(69)
<400> 40
   aaa gtg gag cat tca gac ttg tct ttc tct tgg gac tgg tct ttc tat      48
   Lys Val Glu His Ser Asp Leu Ser Phe Ser Trp Asp Trp Ser Phe Tyr
   1               5                   10                  15
   ctc ttg tac tac act gaa ttc                                          69
   Leu Leu Tyr Tyr Thr Glu Phe
               20
<210> 41
   <211> 23
   <212> PRT
   <213> Artificial
<220>
   <223> mutant sequence
<400> 41
   Lys Val Glu His Ser Asp Leu Ser Phe Ser Trp Asp Trp Ser Phe Tyr
   1               5                   10                  15
   Leu Leu Tyr Tyr Thr Glu Phe
               20
<210> 42
   <211> 69
   <212> DNA
   <213> Artificial
<220>
   <223> mutant sequence
<400> 42
   gaattcagtg tagtacaaga gatagaaaga ccagtcccaa gagaaagaca agtctgaatg      60
   ctccacttt                                                              69
<210> 43
   <211> 75
   <212> DNA
   <213> Artificial
<220>
   <223> mutant sequence
<220>
   <221> CDS
   <222> (1)..(75)
<400> 43
   aaa gtg gag cat tca gac ttg tct ttc tct ggt cgc ggc gac tgg tct      48
   Lys Val Glu His Ser Asp Leu Ser Phe Ser Gly Arg Gly Asp Trp Ser
   1               5                   10                  15
   ttc tat ctc ttg tac tac act gaa ttc                                  75
   Phe Tyr Leu Leu Tyr Tyr Thr Glu Phe
               20                  25
<210> 44
   <211> 25
   <212> PRT
   <213> Artificial
<220>
   <223> mutant sequence
<400> 44
   Lys Val Glu His Ser Asp Leu Ser Phe Ser Gly Arg Gly Asp Trp Ser
   1               5                   10                  15
   Phe Tyr Leu Leu Tyr Tyr Thr Glu Phe
               20                  25
<210> 45
   <211> 75
   <212> DNA
   <213> Artificial
<220>
   <223> mutant sequence
<400> 45
   gaattcagtg tagtacaaga gatagaaaga ccagtcgccg cgaccagaga aagacaagtc      60
   tgaatgctcc acttt                                                       75
<210> 46
   <211> 75
   <212> DNA
   <213> Artificial
<220>
   <223> mutant sequence
<220>
   <221> CDS
   <222> (1)..(75)
<400> 46
   aaa gtg gag cat tca gac ttg tct ttc agc aag ggt gag ggc tgg tct        48
   Lys Val Glu His Ser Asp Leu Ser Phe Ser Lys Gly Glu Gly Trp Ser
   1               5                   10                  15
   ttc tat ctc ttg tac tac act gaa ttc                                    75
   Phe Tyr Leu Leu Tyr Tyr Thr Glu Phe
               20                  25
<210> 47
   <211> 25
   <212> PRT
   <213> Artificial
<220>
   <223> mutant sequence
<400> 47
   Lys Val Glu His Ser Asp Leu Ser Phe Ser Lys Gly Glu Gly Trp Ser
   1               5                   10                  15
   Phe Tyr Leu Leu Tyr Tyr Thr Glu Phe
               20                  25
<210> 48
   <211> 75
   <212> DNA
   <213> Artificial
<220>
   <223> mutant sequence
<400> 48
   gaattcagtg tagtacaaga gatagaaaga ccagccctca cccttgctga aagacaagtc      60
   tgaatgctcc acttt                                                       75
<210> 49
   <211> 69
   <212> DNA
   <213> Artificial
<220>
   <223> mutant sequence
<220>
   <221> CDS
   <222> (1)..(69)
<400> 49
   aaa gtg gag cat tca gac ttg tct ttc agc aag gac ggt tct ttc tat        48
   Lys Val Glu His Ser Asp Leu Ser Phe Ser Lys Asp Gly Ser Phe Tyr
   1               5                   10                  15
   ctc ttg tac tac act gaa ttc                                            69
   Leu Leu Tyr Tyr Thr Glu Phe
               20
<210> 50
   <211> 23
   <212> PRT
   <213> Artificial
<220>
   <223> mutant sequence
<400> 50
   Lys Val Glu His Ser Asp Leu Ser Phe Ser Lys Asp Gly Ser Phe Tyr
   1               5                   10                  15
   Leu Leu Tyr Tyr Thr Glu Phe
               20
<210> 51
   <211> 69
   <212> DNA
   <213> Artificial
<220>
   <223> mutant sequence
<400> 51
   gaattcagtg tagtacaaga gatagaaaga accgtccttg ctgaaagaca agtctgaatg    60
   ctccacttt                                                            69
<210> 52
   <211> 69
   <212> DNA
   <213> Artificial
<220>
   <223> mutant sequence
<220>
   <221> CDS
   <222> (1)..(69)
<400> 52
   aaa gtg gag cat tca gac ttg tct ttc tct cgc gac ggt tct ttc tat      48
   Lys Val Glu His Ser Asp Leu Ser Phe Ser Arg Asp Gly Ser Phe Tyr
   1               5                   10                  15
   ctc ttg tac tac act gaa ttc                                          69
   Leu Leu Tyr Tyr Thr Glu Phe
               20
<210> 53
   <211> 23
   <212> PRT
   <213> Artificial
<220>
   <223> mutant sequence
<400> 53
   Lys Val Glu His Ser Asp Leu Ser Phe Ser Arg Asp Gly Ser Phe Tyr
   1               5                   10                  15
   Leu Leu Tyr Tyr Thr Glu Phe
               20
<210> 54
   <211> 69
   <212> DNA
   <213> Artificial
<220>
   <223> mutant sequence
<400> 54
   gaattcagtg tagtacaaga gatagaaaga accgtcgcga gagaaagaca agtctgaatg      60
   ctccacttt                                                              69
<210> 55
   <211> 69
   <212> DNA
   <213> Artificial
<220>
   <223> mutant sequence
<220>
   <221> CDS
   <222> (1)..(69)
<400> 55
   aaa gtg gag cat tca gac ttg tct ttc tct gac gac tgg tct ttc tat        48
   Lys Val Glu His Ser Asp Leu Ser Phe Ser Asp Asp Trp Ser Phe Tyr
   1               5                   10                  15
   ctc ttg tac tac act gaa ttc                                            69
   Leu Leu Tyr Tyr Thr Glu Phe
               20
<210> 56
   <211> 23
   <212> PRT
   <213> Artificial
<220>
   <223> mutant sequence
<400> 56
   Lys Val Glu His Ser Asp Leu Ser Phe Ser Asp Asp Trp Ser Phe Tyr
   1               5                   10                  15
   Leu Leu Tyr Tyr Thr Glu Phe
               20
<210> 57
   <211> 69
   <212> DNA
   <213> Artificial
<220>
   <223> mutant sequence
<400> 57
   gaattcagtg tagtacaaga gatagaaaga ccagtcctca gagaaagaca agtctgaatg 60
   ctccacttt 69
<210> 58
   <211> 47
   <212> DNA
   <213> Artificial
<220>
   <223> plasmid sequence
<400> 58
   taatacgact cactataggc tagcctcgag aattcacgcg tcgagca 47
<210> 59
   <211> 59
   <212> DNA
   <213> Artificial
<220>
   <223> plasmid sequence
<400> 59
   caacccggga tcctctagag tcgacccggg cggccgcttc cctttcgtga gggttaatg 59
<210> 60
   <211> 1098
   <212> DNA
   <213> Homo sapiens
<400> 60
   atggccgtca tggcgccccg aaccctcgtc ctgctactct cgggggctct ggccctgacc     60
   cagacctggg cgggctctca ctccatgagg tatttcttca catccgtgtc ccggcccggc    120
   cgcggggagc cccgcttcat cgcagtgggc tacgtggacg acacgcagtt cgtgcggttc    180
   gacagcgacg ccgcgagcca gaggatggag ccgcgggcgc cgtggataga gcaggagggt    240
   ccggagtatt gggacgggga gacacggaaa gtgaaggccc actcacagac tcaccgagtg    300
   gacctgggga ccctgcgcgg ctactacaac cagagcgagg ccggttctca caccgtccag    360
   aggatgtatg gctgcgacgt ggggtcggac tggcgcttcc tccgcgggta ccaccagtac    420
   gcctacgacg gcaaggatta catcgccctg aaagaggacc tgcgctcttg gaccgcggcg    480
   gacatggcag ctcagaccac caagcacaag tgggaggcgg cccatgtggc ggagcagttg    540
   agagcctacc tggagggcac gtgcgtggag tggctccgca gatacctgga gaacgggaag    600
   gagacgctgc agcgcacgga cgcccccaaa acgcatatga ctcaccacgc tgtctctgac    660
   catgaagcca ccctgaggtg ctgggccctg agcttctacc ctgcggagat cacactgacc    720
   tggcagcggg atggggagga ccagacccag gacacggagc tcgtggagac caggcctgca    780
   ggggatggaa ccttccagaa gtgggcggct gtggtggtgc cttctggaca ggagcagaga    840
   tacacctgcc atgtgcagca tgagggtttg cccaagcccc tcaccctgag atgggagccg    900
   tcttcccagc ccaccatccc catcgtgggc atcattgctg gcctggttct ctttggagct    960
   gtgatcactg gagctgtggt cgctgctgtg atgtggagga ggaagagctc agatagaaaa   1020
   ggagggagct actctcaggc tgcaagcagt gacagtgccc agggctctga tgtgtctctc   1080
   acagcttgta aagtgtga                                                 1098
<210> 61
   <211> 1098
   <212> DNA
   <213> Homo sapiens
<400> 61
   tcacacttta caagctgtga gagacacatc agagccctgg gcactgtcac tgcttgcagc     60
   ctgagagtag ctccctcctt ttctatctga gctcttcctc ctccacatca cagcagcgac    120
   cacagctcca gtgatcacag ctccaaagag aaccaggcca gcaatgatgc ccacgatggg    180
   gatggtgggc tgggaagacg gctcccatct cagggtgagg ggcttgggca aaccctcatg    240
   ctgcacatgg caggtgtatc tctgctcctg tccagaaggc accaccacag ccgcccactt    300
   ctggaaggtt ccatcccctg caggcctggt ctccacgagc tccgtgtcct gggtctggtc    360
   ctccccatcc cgctgccagg tcagtgtgat ctccgcaggg tagaagctca gggcccagca    420
   cctcagggtg gcttcatggt cagagacagc gtggtgagtc atatgcgttt tgggggcgtc    480
   cgtgcgctgc agcgtctcct tcccgttctc caggtatctg cggagccact ccacgcacgt    540
   gccctccagg taggctctca actgctccgc cacatgggcc gcctcccact tgtgcttggt    600
   ggtctgagct gccatgtccg ccgcggtcca agagcgcagg tcctctttca gggcgatgta    660
   atccttgccg tcgtaggcgt actggtggta cccgcggagg aagcgccagt ccgaccccac    720
   gtcgcagcca tacatcctct ggacggtgtg agaaccggcc tcgctctggt tgtagtagcc    780
   gcgcagggtc cccaggtcca ctcggtgagt ctgtgagtgg gccttcactt tccgtgtctc    840
   cccgtcccaa tactccggac cctcctgctc tatccacggc gcccgcggct ccatcctctg    900
   gctcgcggcg tcgctgtcga accgcacgaa ctgcgtgtcg tccacgtagc ccactgcgat    960
   gaagcggggc tccccgcggc cgggccggga cacggatgtg aagaaatacc tcatggagtg   1020
   agagcccgcc caggtctggg tcagggccag agcccccgag agtagcagga cgagggttcg   1080
   gggcgccatg acggccat                                                 1098
<210> 62
   <211> 360
   <212> DNA
   <213> Homo sapiens
<400> 62
   atgtctcgct ccgtggcctt agctgtgctc gcgctactct ctctttctgg cctggaggct     60
   atccagcgta ctccaaagat tcaggtttac tcacgtcatc cagcagagaa tggaaagtca    120
   aatttcctga attgctatgt gtctgggttt catccatccg acattgaagt tgacttactg    180
   aagaatggag agagaattga aaaagtggag cattcagact tgtctttcag caaggactgg    240
   tctttctatc tcttgtacta cactgaattc acccccactg aaaaagatga gtatgcctgc    300
   cgtgtgaacc atgtgacttt gtcacagccc aagatagtta agtgggatcg agacatgtaa    360
<210> 63
   <211> 360
   <212> DNA
   <213> Homo sapiens
<400> 63
   ttacatgtct cgatcccact taactatctt gggctgtgac aaagtcacat ggttcacacg      60
   gcaggcatac tcatcttttt cagtgggggt gaattcagtg tagtacaaga gatagaaaga     120
   ccagtccttg ctgaaagaca agtctgaatg ctccactttt tcaattctct ctccattctt     180
   cagtaagtca acttcaatgt cggatggatg aaacccagac acatagcaat tcaggaaatt     240
   tgactttcca ttctctgctg gatgacgtga gtaaacctga atctttggag tacgctggat     300
   agcctccagg ccagaaagag agagtagcgc gagcacagct aaggccacgg agcgagacat     360
<210> 64
   <211> 3473
   <212> DNA
   <213> Artificial
<220>
   <223> plasmid sequence
<220>
   <221> CDS
   <222> (100)..(477)
<400> 64
   gatctcgatc ccgcgaaatt aatacgactc actataggga gaccacaacg gtttccctct      60
   agaaataatt ttgtttaact ttaagaagga gatatacat atg ggt att tta gga       114
                                              Met Gly Ile Leu Gly
                                              1               5
   ttt gtt ttt aca tta gga tcc ggt ggg gga ggc gga tca gga ggc tca       162
   Phe Val Phe Thr Leu Gly Ser Gly Gly Gly Gly Gly Ser Gly Gly Ser
                   10                  15                  20
   ggt ggg tca gga ggc atc cag cgt act cca aag att cag gtt tac tca       210
   Gly Gly Ser Gly Gly Ile Gln Arg Thr Pro Lys Ile Gln Val Tyr Ser
               25                  30                  35
   cgt cat cca gca gag aat gga aag tca aat ttc ctg aat tgc tat gtg       258
   Arg His Pro Ala Glu Asn Gly Lys Ser Asn Phe Leu Asn Cys Tyr Val
           40                  45                  50
   tct ggg ttt cat cca tcc gac att gaa gtt gac tta ctg aag aat gga       306
   Ser Gly Phe His Pro Ser Asp Ile Glu Val Asp Leu Leu Lys Asn Gly
       55                  60                  65
   gag aga att gaa aaa gtg gag cat tca gac ttg tct ttc agc aag gac       354
   Glu Arg Ile Glu Lys Val Glu His Ser Asp Leu Ser Phe Ser Lys Asp
   70                  75                  80                  85
   tgg tct ttc tat ctc ttg tac tac act gaa ttc acc ccc act gaa aaa       402
   Trp Ser Phe Tyr Leu Leu Tyr Tyr Thr Glu Phe Thr Pro Thr Glu Lys
                   90                  95                  100
   gat gag tat gcc tgc cgt gtg aac cat gtg act ttg tca cag ccc aag       450
   Asp Glu Tyr Ala Cys Arg Val Asn His Val Thr Leu Ser Gln Pro Lys
               105                 110                 115
   ata gtt aag tgg gat cga gac atg taa gcttgaattc cgatccggct             497
   Ile Val Lys Trp Asp Arg Asp Met
           120                 125
   gctaacaaag cccgaaagga agctgagttg gctgctgcca ccgctgagca ataactagca     557
   taaccccttg gggcctctaa acgggtcttg aggggttttt tgctgaaagg aggaactata     617
   tccggataat tcttgaagac gaaagggcct cgtgatacgc ctatttttat aggttaatgt     677
   catgataata atggtttctt agacgtcagg tggcactttt cggggaaatg tgcgcggaac     737
   ccctatttgt ttatttttct aaatacattc aaatatgtat ccgctcatga gacaataacc     797
   ctgataaatg cttcaataat attttgttaa aattcgcgtt aaatttttgt taaatcagct     857
   cattttttaa ccaataggcc gaaatcggca aaatccctta taaatcaaaa gaatagaccg      917
   agatagggtt gagtgttgtt ccagtttgga acaagagtcc actattaaag aacgtggact     977
   ccaacgtcaa agggcgaaaa accgtctatc agggcgatgg cccactacgt gaaccatcac 1037
   cctaatcaag ttttttgggg tcgaggtgcc gtaaagcact aaatcggaac cctaaaggga 1097
   gcccccgatt tagagcttga cggggaaagc cggcgaacgt ggcgagaaag gaagggaaga 1157
   aagcgaaagg agcgggcgct agggcgctgg caagtgtagc ggtcacgctg cgcgtaacca 1217
   ccacacccgc cgcgcttaat gcgccgctac agggcgcgtc aggtggcact tttcggggaa 1277
   atgtgcgcgg aacccctatt tgtttatttt tctaaataca ttcaaatatg tatccgctca 1337
   tgagacaata accctgataa atgcttcaat aatattgaaa aaggaagagt atgagtattc 1397
   aacatttccg tgtcgccctt attccctttt ttgcggcatt ttgccttcct gtttttgctc 1457
   acccagaaac gctggtgaaa gtaaaagatg ctgaagatca gttgggtgca cgagtgggtt 1517
   acatcgaact ggatctcaac agcggtaaga tccttgagag ttttcgcccc gaagaacgtt 1577
   ttccaatgat gagcactttt aaagttctgc tatgtggcgc ggtattatcc cgtgttgacg 1637
   ccgggcaaga gcaactcggt cgccgcatac actattctca gaatgacttg gttgagtact 1697
   caccagtcac agaaaagcat cttacggatg gcatgacagt aagagaatta tgcagtgctg 1757
   ccataaccat gagtgataac actgcggcca acttacttct gacaacgatc ggaggaccga 1817
   aggagctaac cgcttttttg cacaacatgg gggatcatgt aactcgcctt gatcgttggg 1877
   aaccggagct gaatgaagcc ataccaaacg acgagcgtga caccacgatg cctgcagcaa 1937
   tggcaacaac gttgcgcaaa ctattaactg gcgaactact tactctagct tcccggcaac 1997
   aattaataga ctggatggag gcggataaag ttgcaggacc acttctgcgc tcggcccttc 2057
   cggctggctg gtttattgct gataaatctg gagccggtga gcgtgggtct cgcggtatca 2117
   ttgcagcact ggggccagat ggtaagccct cccgtatcgt agttatctac acgacgggga 2177
   gtcaggcaac tatggatgaa cgaaatagac agatcgctga gataggtgcc tcactgatta 2237
   agcattggta actgtcagac caagtttact catatatact ttagattgat ttaaaacttc 2297
   atttttaatt taaaaggatc taggtgaaga tcctttttga taatctcatg accaaaatcc 2357
   cttaacgtga gttttcgttc cactgagcgt cagaccccgt agaaaagatc aaaggatctt 2417
   cttgagatcc tttttttctg cgcgtaatct gctgcttgca aacaaaaaaa ccaccgctac 2477
   cagcggtggt ttgtttgccg gatcaagagc taccaactct ttttccgaag gtaactggct 2537
   tcagcagagc gcagatacca aatactgtcc ttctagtgta gccgtagtta ggccaccact 2597
   tcaagaactc tgtagcaccg cctacatacc tcgctctgct aatcctgtta ccagtggctg 2657
   ctgccagtgg cgataagtcg tgtcttaccg ggttggactc aagacgatag ttaccggata 2717
   aggcgcagcg gtcgggctga acggggggtt cgtgcacaca gcccagcttg gagcgaacga 2777
   cctacaccga actgagatac ctacagcgtg agctatgaga aagcgccacg cttcccgaag 2837
   ggagaaaggc ggacaggtat ccggtaagcg gcagggtcgg aacaggagag cgcacgaggg 2897
   agcttccagg gggaaacgcc tggtatcttt atagtcctgt cgggtttcgc cacctctgac 2957
   ttgagcgtcg atttttgtga tgctcgtcag gggggcggag cctatggaaa aacgccagca 3017
   acgcggcctt tttacggttc ctggcctttt gctggccttt tgctcacatg ttctttcctg 3077
   cgttatcccc tgattctgtg gataaccgta ttaccgcctt tgagtgagct gataccgctc 3137
   gccgcagccg aacgaccgag cgcagcgagt cagtgagcga ggaagcggaa gagcgcctga 3197
   tgcggtattt tctccttacg catctgtgcg gtatttcaca ccgcaatggt gcactctcag 3257
   tacaatctgc tctgatgccg catagttaag ccagtataca ctccgctatc gctacgtgac 3317
   tgggtcatgg ctgcgccccg acacccgcca acacccgctg acgcgccctg acgggcttgt 3377
   ctgctcccgg catccgctta cagacaagct gtgaccgtct ccgggagctg catgtgtcag 3437
   aggttttcac cgtcatcacc gaaacgcgcg aggcag 3473
<210> 65
   <211> 125
   <212> PRT
   <213> Artificial
<220>
   <223> plasmid sequence
<400> 65
   Met Gly Ile Leu Gly Phe Val Phe Thr Leu Gly Ser Gly Gly Gly Gly
   1               5                   10                  15
   Gly Ser Gly Gly Ser Gly Gly Ser Gly Gly Ile Gln Arg Thr Pro Lys
               20                  25                  30
   Ile Gln Val Tyr Ser Arg His Pro Ala Glu Asn Gly Lys Ser Asn Phe
           35                  40                  45
   Leu Asn Cys Tyr Val Ser Gly Phe His Pro Ser Asp Ile Glu Val Asp
       50                  55                  60
   Leu Leu Lys Asn Gly Glu Arg Ile Glu Lys Val Glu His Ser Asp Leu
   65                  70                  75                  80
   Ser Phe Ser Lys Asp Trp Ser Phe Tyr Leu Leu Tyr Tyr Thr Glu Phe
                   85                  90                  95
   Thr Pro Thr Glu Lys Asp Glu Tyr Ala Cys Arg Val Asn His Val Thr
               100                 105                 110
   Leu Ser Gln Pro Lys Ile Val Lys Trp Asp Arg Asp Met
           115                 120                 125